# EUROPEAN PATENT APPLICATION

(11) **EP 4 365 204 A1**
(43) Date of publication of application: **08.05.2024**
(21) Application number: 22833232.6
(22) Date of filing: 29.06.2022
(51) Int. Cl.: C07K 16/30, C12N 5/078, C12N 5/0784, C12N 5/0786, C12N 5/09, C12N 15/10, C12Q 1/6806, C12Q 1/6869, G01N 33/50

(54) **METHOD FOR PREPARING SAMPLE CONTAINING TUMOR CELLS CIRCULATING IN BLOOD**

(30) Priority: 30.06.2021 JP 2021108784
(71) Applicant: Novacellum Inc., Tokyo 102-0082 (JP)
(72) Inventor: YAMASHITA, Naohide, Tokyo 177-0042 (JP)
(74) Representative: Vossius & Partner Patentanwälte Rechtsanwälte mbB
(86) International application number: PCT/JP2022/026036
(87) International publication number: WO 2023/277086

(57) **Abstract**

According to the present disclosure, a neoantigen is identified from a blood sample of a patient, or a sample for the identification is provided. According to the present disclosure, there is provided a method for preparing a sample containing circulating tumor cells derived from a subject for analysis without nucleic acid amplification and/or proliferation by culture, the method including the steps of: isolating monocytes from peripheral blood mononuclear cells collected from the subject using apheresis, wherein apheresis condition for performing the apheresis includes circulating tumor cell enrichment condition; isolating circulating tumor cells from the peripheral blood mononuclear cells from which the monocytes have been isolated; optionally, confirming a purity of circulating tumor cells required for the analysis; and optionally, performing pretreatment for the analysis on the circulating tumor cells.

## Description

### Technical Field

The present disclosure relates to a method for preparing a sample containing circulating tumor cells derived from a subject for analysis without proliferation, and a method for analyzing circulating tumor cells derived from a subject without proliferation. In particular, the present disclosure relates to a method for preparing a sample for identifying a neoantigen possessed by a subject, a method for identifying a neoantigen possessed by a subject from the sample, and a method for producing a cell for dendritic cell therapy using a neoantigen possessed by a subject.

### Background Art

Currently, cancer immunotherapy using neoantigens has attracted attention as next-generation immunotherapy of immune checkpoint inhibitors. Neoantigens, also called neo-antigens or neoantigens, are mutated antigens that have newly appeared due to genetic mutations occurring in cancer cells. Since the neoantigens vary depending on individual patients, application to personalized medicine is expected. In addition, it is known that neoantigens newly generated in cancer tissues cause a strong immune reaction, and it is also suggested that neoantigens are well compatible with cancer immunotherapy such as an immune checkpoint inhibitor.

### Summary of Invention

### Technical Problem

The present inventors have found a technique for identifying a neoantigen from a patient's blood sample without using an amount of tumor tissue conventionally required as a sample for neoantigen identification. In addition, the present inventors have found a technique for preparing a sample that can be used for analysis other than neoantigen identification from a patient's blood sample.

Accordingly, the present disclosure provides the following inventions.

### (Item X1)

A method for preparing a sample containing circulating tumor cells derived from a subject for analysis without nucleic acid amplification and/or proliferation by culture, the method including the steps of:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
optionally, confirming a purity of circulating tumor cells required for the analysis; and
optionally, performing pretreatment for the analysis on the circulating tumor cells.

### (Item X2)

The method according to the preceding item, wherein the apheresis condition includesubjecting a spillover volume and/or a buffy coat volume to the circulating tumor cell enrichment condition.

### (Item X3)

The method according to any one of the preceding items, wherein the apheresis condition includes a spillover volume of about 9 to about 12 ml and/or a buffy coat volume of about 7 to about 10 ml.

### (Item X4)

The method according to any one of the preceding items, wherein the step of isolating circulating tumor cells is carried out using a monoclonal antibody or by size fractionation.

### (Item X5)

The method according to any one of the preceding items, wherein a number of the circulating tumor cells is about 1 × 10³ or more.

### (Item X6)

The method according to any one of the preceding items, wherein a number of the peripheral blood mononuclear cells is about 1 × 10⁴ or more.

### (Item X7)

The method according to any one of the preceding items, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

### (Item X8)

The method according to any one of the preceding items, further including a step of freezing the circulating tumor cells.

### (Item X9)

The method according to any one of the preceding items, wherein the analysis includes whole exome analysis, whole genome analysis, RNA-Seq, single cell RNA-Seq, proteome analysis, and transcriptome analysis.

### (Item X10)

The method according to any one of the preceding items, wherein the sample is for identifying a neoantigen possessed by the subject by performing the analysis.

### (Item X11)

The method according to any one of the preceding items, further including a step of extracting DNA, RNA, or protein from the circulating tumor cells.

### (Item X12)

The method according to any one of the preceding items, wherein the DNA or RNA is extracted at least in an amount of about 100 pg.

### (Item X13)

A method for analyzing circulating tumor cells derived from a subject without nucleic acid amplification and/or proliferation by culture, the method including the steps of:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated; and
performing analysis using the circulating tumor cells.

### (Item X14)

The method according to any one of the preceding items, wherein the apheresis condition includesubjecting a spillover volume and/or a buffy coat volume to the circulating tumor cell enrichment condition.

### (Item X15)

The method according to any one of the preceding items, wherein the apheresis condition includes a spillover volume of about 9 to about 12 ml and/or a buffy coat volume of about 7 to about 10 ml.

### (Item X16)

The method according to any one of the preceding items, wherein the step of isolating circulating tumor cells is carried out using a monoclonal antibody or by size fractionation.

### (Item X17)

The method according to any one of the preceding items, wherein a number of the circulating tumor cells is about 1 × 10³ or more.

### (Item X18)

The method according to any one of the preceding items, wherein a number of the peripheral blood mononuclear cells is about 1 × 10⁴ or more.

### (Item X19)

The method according to any one of the preceding items, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

### (Item X20)

The method according to any one of the preceding items, further including a step of freezing the circulating tumor cells.

### (Item X21)

The method according to any one of the preceding items, wherein the analysis includes whole exome analysis, whole genome analysis, RNA-Seq, single cell RNA-Seq, proteome analysis, and transcriptome analysis.

### (Item X22)

A method for identifying a neoantigen possessed by the subject by analyzing the circulating tumor cells derived from the subject by the method according to any one of the preceding items.

### (Item X23)

The method according to any one of the preceding items, further including a step of extracting DNA, RNA, or protein from the circulating tumor cells.

### (Item X24)

The method according to any one of the preceding items, further including the steps of:
creating a DNA/RNA sequence library from the DNA or RNA; and
identifying a neoantigen based on mutation information in the sequence library,
wherein the circulating tumor cells have a purity of at least about 20%.

### (Item X25)

The method according to any one of the preceding items, wherein the analysis includes a whole exome analysis of the DNA.

### (Item X26)

The method according to any one of the preceding items, wherein the analysis further includes proteomic analysis of the circulating tumor cells.

### (Item X27)

The method according to any one of the preceding items, wherein the DNA or RNA is extracted at least in an amount of about 100 pg.

### (Item X28)

A method for producing a cell for dendritic cell therapy using a neoantigen possessed by a subject, the method including:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
extracting DNA or RNA from the circulating tumor cell to create a DNA/RNA sequence library;
identifying a neoantigen based on mutation information of the sequence library; and
introducing the neoantigen into dendritic cells.

### (Item X29)

The method according to any one of the preceding items, wherein the apheresis condition includesubjecting a spillover volume and/or a buffy coat volume to the circulating tumor cell enrichment condition.

### (Item X30)

The method according to any one of the preceding items, wherein the apheresis condition includes a spillover volume of about 9 to about 12 ml and/or a buffy coat volume of about 7 to about 10 ml.

### (Item X31)

The method according to any one of the preceding items, wherein the step of isolating circulating tumor cells is carried out using a monoclonal antibody or by size fractionation.

### (Item X32)

The method according to any one of the preceding items, wherein a number of the circulating tumor cells is about 1 × 10³ or more.

### (Item X33)

The method according to any one of the preceding items, wherein a number of the peripheral blood mononuclear cells is about 1 × 10⁴ or more.

### (Item X34)

The method according to any one of the preceding items, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

### (Item X35)

The method according to any one of the preceding items, further including a step of freezing the circulating tumor cells.

### (Item X36)

The method according to any one of the preceding items, wherein the DNA or RNA is extracted at least in an amount of about 100 pg.

### (Item X37)

The method according to any one of the preceding items, wherein the dendritic cells are obtained from the subject by the apheresis or derived from the monocytes.

### (Item X38)

A method for treating or preventing a subject by analyzing circulating tumor cells without nucleic acid amplification and/or proliferation by culture, the method including the steps of:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
performing analysis using the circulating tumor cells; and
treating or preventing the subject based on results of the analysis.

### (Item X39)

The method according to any one of the preceding items, wherein the analysis includes whole exome analysis, whole genome analysis, RNA-Seq, single cell RNA-Seq, proteome analysis, and transcriptome analysis.

The present disclosure also provides the following inventions.

### (Item 1)

A method for preparing a sample for identifying a neoantigen possessed by a subject by sequencing without nucleic acid amplification and/or proliferation by culture, the method including the steps of:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
optionally, confirming a purity of circulating tumor cells required for the identification of the neoantigen; and
providing the circulating tumor cells as a sample for identifying the neoantigen and/or extracting DNA and/or RNA from the circulating tumor cells.

### (Item 2)

The method according to the preceding item, wherein the apheresis condition includesubjecting a spillover volume and/or a buffy coat volume to the circulating tumor cell enrichment condition.

### (Item 3)

The method according to any one of the preceding items, wherein the apheresis condition includes a spillover volume of about 9 to about 12 ml and/or a buffy coat volume of about 7 to about 10 ml.

### (Item 4)

The method according to any one of the preceding items, wherein the step of isolating circulating tumor cells is carried out using a monoclonal antibody or by size fractionation.

### (Item 5)

The method according to any one of the preceding items, wherein a number of the circulating tumor cells is about 1 × 10³ or more.

### (Item 6)

The method according to any one of the preceding items, wherein a number of the peripheral blood mononuclear cells is about 1 × 10⁴ or more.

### (Item 7)

The method according to any one of the preceding items, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

### (Item 8)

The method according to any one of the preceding items, further including a step of freezing the circulating tumor cells.

### (Item 9)

The method according to any one of the preceding items, wherein the DNA or RNA is extracted at least in an amount of about 100 pg.

### (Item A1)

A method for preparing a sample for identifying a neoantigen possessed by a subject by sequencing without nucleic acid amplification and/or proliferation by culture, the method including the steps of:
isolating monocytes from peripheral blood mononuclear cells collected from the subject using apheresis;
isolating circulating tumor cells from the peripheral blood mononuclear cells from which the monocytes have been isolated;
optionally, confirming a purity of circulating tumor cells required for the identification of the neoantigen; and
providing the circulating tumor cells as a sample for identifying the neoantigen and/or extracting DNA and/or RNA from the circulating tumor cells.

### (Item B1)

A method for identifying a neoantigen possessed by a subject by sequencing without nucleic acid amplification and/or expansion by culture, the method including:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
extracting DNA and/or RNA from the circulating tumor cell to create a DNA/RNA sequence library; and
identifying a neoantigen based on mutation information of the sequence library,
wherein the circulating tumor cells have a purity of at least about 20%.

### (Item B2)

The method according to any one of the preceding items, wherein the apheresis condition includesubjecting a spillover volume and/or a buffy coat volume to the circulating tumor cell enrichment condition.

### (Item B3)

The method according to any one of the preceding items, wherein the apheresis condition includes a spillover volume of about 9 to about 12 ml and/or a buffy coat volume of about 7 to about 10 ml.

### (Item B4)

The method according to any one of the preceding items, wherein the step of isolating circulating tumor cells is carried out using a monoclonal antibody or by size fractionation.

### (Item B5)

The method according to any one of the preceding items, wherein a number of the circulating tumor cells is about 1 × 10³ or more.

### (Item B6)

The method according to any one of the preceding items, wherein a number of the peripheral blood mononuclear cells is about 1 × 10⁴ or more.

### (Item B7)

The method according to any one of the preceding items, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

### (Item B8)

The method according to any one of the preceding items, further including a step of freezing the circulating tumor cells.

### (Item B9)

The method according to any one of the preceding items, wherein the DNA or RNA is extracted at least in an amount of about 100 pg.

### (Item C1)

A method for producing a cell for dendritic cell therapy using a neoantigen possessed by a subject, the method including:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
extracting DNA and/or RNA from the circulating tumor cell to create a DNA/RNA sequence library;
identifying a neoantigen based on mutation information of the sequence library; and
introducing the neoantigen into dendritic cells.

### (Item C2)

The method according to any one of the preceding items, wherein the apheresis condition includesubjecting a spillover volume and/or a buffy coat volume to the circulating tumor cell enrichment condition.

### (Item C3)

The method according to any one of the preceding items, wherein the apheresis condition includes a spillover volume of about 9 to about 12 ml and/or a buffy coat volume of about 7 to about 10 ml.

### (Item C4)

The method according to any one of the preceding items, wherein the step of isolating circulating tumor cells is carried out using a monoclonal antibody or by size fractionation.

### (Item C5)

The method according to any one of the preceding items, wherein a number of the circulating tumor cells is about 1 × 10³ or more.

### (Item C6)

The method according to any one of the preceding items, wherein a number of the peripheral blood mononuclear cells is about 1 × 10⁴ or more.

### (Item C7)

The method according to any one of the preceding items, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

### (Item C8)

The method according to any one of the preceding items, further including a step of freezing the circulating tumor cells.

### (Item C9)

The method according to any one of the preceding items, wherein the DNA or RNA is extracted at least in an amount of about 100 pg.

### (Item C10)

The method according to any one of the preceding items, wherein the dendritic cells are obtained from the subject by the apheresis or derived from the monocytes.

In the present disclosure, it is intended that the one or more features described above may be provided in further combinations in addition to the specified combinations. It should be noted that further embodiments and advantages of the present disclosure will be appreciated by those skilled in the art upon reading and understanding the following detailed description as appropriate.

Note that features and remarkable actions and effects of the present disclosure other than those described above will be apparent to those skilled in the art with reference to the following embodiments and drawings of the invention. Advantageous Effects of Invention

According to the method of the present disclosure, a neoantigen of a patient can be non-invasively identified. The method of the present disclosure can be helpful for cancer immunotherapy using neoantigens, and can also be developed into uses in personalized medicine and regenerative medicine.

### Brief Description of Drawings

FIG. 1 is a schematic view showing a procedure for making neoantigen dendritic cells according to one embodiment of the present disclosure and a flow of dendritic cell therapy using the same.
FIG. 2 is a view showing an analysis result by flow cytometry of cells isolated in one embodiment of the present disclosure.
FIG. 3 is a schematic view showing a procedure for making neoantigen dendritic cells according to another embodiment of the present disclosure and a flow of dendritic cell therapy using the same.
FIG. 4 is a view showing an analysis result by flow cytometry of cells isolated in one embodiment of the present disclosure. Results are shown for NY lung cancer (stage 4) patients, CTC 20-0065 colorectal cancer (stage 4) patients, and C-0107 ovarian cancer (stage 4) patients.
FIG. 5 is a view showing an analysis result by flow cytometry of cells isolated in one embodiment of the present disclosure. Results are shown for 21-0062 breast cancer patients (post-operative), CTC 20-014 pancreatic cancer (stage 4) patients, and C-0082 pancreatic cancer (stage 4) patients.
FIG. 6 is a view showing an analysis result by flow cytometry of cells isolated in one embodiment of the present disclosure. Results are shown for 21-0059 duodenal papilla cancer (stage 4) patients and CTC 21-0052 lung cancer (stage 4) patients.
FIG. 7 is a view showing an analysis result by flow cytometry of cells isolated in one embodiment of the present disclosure. Results are shown for CTC 20-008 lung cancer (stage 4) patients (treatment successful), 21-0060 colorectal cancer (post-operative), and 21-0063 colorectal cancer (stage 4) patients (elderly).
FIG. 8 is a view showing an analysis result by flow cytometry of cells isolated in one embodiment of the present disclosure. Results are shown for CTC 20-019 pancreatic cancer (stage 2B) patients, ID 10277 liver cancer (multiple liver cancer) patients (CR after treatment and then small recurrence in the liver), and T-0014 prostate cancer (stage 4) patients (hormone treatment successful and significant decrease in PSA from 216.88 to 4.91).
FIG. 9 is a view showing an analysis result by flow cytometry of cells isolated in one embodiment of the present disclosure. Results for C-0099 (esophageal cancer) (post-operative) are shown.

### Description of Embodiments

Hereinafter, the present disclosure will be described while showing the best mode. It is to be understood that throughout the specification, expressions in the singular form also encompass concepts thereof in the plural form, unless otherwise stated. Thus, it is to be understood that terms attached with singular articles (for example, in English, "a", "an", and "the") also encompass concepts thereof in the plural form, unless otherwise stated. It is also to be understood that the terms used herein are used in the sense commonly used in the art, unless otherwise stated. Thus, unless otherwise defined, all technical and scientific terms used herein have the same meanings as commonly understood by those skilled in the art to which the present disclosure pertains. In case of conflict, the present specification (including definitions) will control.

Hereinafter, definitions and/or basic technical contents of terms particularly used in the present specification will be described as appropriate.

As used herein, "about" means ±10% of a numerical value following the term.

As used herein, "neoantigen" is also referred to as a neo-antigen, a neoantigen, or a tumor-specific mutated antigen, and refers to an antigen generated by a DNA mutation that alters a protein in a tumor cell. The neoantigen can potentially be perceived as non-self by the immune system.

In the present specification, the term "apheresis" generally refers to separating one or more blood components (cell components such as plasma components, platelets, and white blood cells) from blood of a subject by extracorporeal circulation. Blood collected by extracorporeal circulation is referred to as apheresis blood. In the present specification, peripheral blood mononuclear cells are collected by extracorporeally circulating blood of a subject by apheresis and passing the blood through a blood component separation device.

In the present specification, "buffy coat" refers to a white layer between red blood cells and plasma after whole blood is precipitated by centrifugation. The whole blood is separated, by centrifugation, into plasma (platelet-poor plasma) as a light specific gravity component (supernatant component), red blood cells (concentrated red blood cells) as a heavy specific gravity component (sedimentation component), and buffy coat as a medium specific gravity component. The buffy coat contains a white blood cell component and platelet-rich plasma (platelet-containing component). "Buffy coat volume" refers to an amount of the buffy coat to be collected.

In the present specification, "spillover" means that, when the buffy coat is sufficiently enriched in apheresis, retransfusion and collection are switched to collect the buffy coat. Excess platelets and lymphocytes can be removed by spillover. "Spillover volume" refers to an amount of the buffy coat to be sent to a collection stage.

In the present specification, when referring to an apheresis condition, "circulating tumor cell enrichment condition" refers to a specific condition for performing apheresis, under which circulating tumor cells are enriched in a resulting section. Typically, the circulating tumor cell enrichment condition can includespillover volume, buffy coat volume, and combinations thereof, and can be achieved by subjecting these values to specific condition. In order to achieve the circulating tumor cell enrichment condition, the spillover volume may advantageously be about 5 to about 15 ml, preferably about 7 to 14 ml, and more preferably about 9 to about 12 ml. In order to achieve the circulating tumor cell enrichment condition, the buffy coat volume may be about 5 to about 15 ml, preferably about 6 to 12 ml, and more preferably about 7 to about 10 ml. In order to achieve the circulating tumor cell enrichment condition, the spillover volume and the buffy coat volume may further advantageously be used under a combination of the above preferred condition.

As used herein, "peripheral blood mononuclear cell", also referred to as PBMC, refers to any peripheral blood cell having a circular nucleus. Normal peripheral blood mononuclear cells consist of lymphocytes (T cells, B cells, NK cells) and monocytes. In the present disclosure, it has been found for the first time that CTCs are substantially contained in a peripheral blood mononuclear cell component.

In the present specification, the "monocyte" is a type of white blood cell, and is the largest type of white blood cell. It refers to a macrophage or a cell capable of differentiating into a dendritic cell. the monocyte also influences the process of adaptive immunity as a part of the innate immunity of vertebrates. It is said that there are at least three types of monocytes in human blood.

As used herein, "circulating tumor cell", also referred to as CTCs, refers to a circulating tumor cell circulating in blood, among tumor cells that have penetrated into blood vessels or lymphatic vessels from a primary tumor (primary focus) or a metastatic tumor (metastatic focus). CTCs exist, for example, as tumor cells circulating in the peripheral blood stream of a cancer patient. CTCs are blood-based markers thought to have predictive and prognostic functions in cancer detection and progression. Many types of cancers are known to produce CTCs. CTCs are nucleated, CD45-negative, and cytokeratin-positive cells, and at least one of a tumor marker, a sarcoma marker, and an osteosarcoma marker is expressed. Isolation of CTCs from whole blood is technically challenging due to a very low number of CTCs.

As used herein, "dendritic cell", also referred to as DC, refers to an antigen presenting cell that is potent for initiation and control of cellular immune responses in humans. Dendritic cells may be either immunostimulatory or immunosuppressive depending on which set of potential properties they express upon interaction with response-specific clones of T cells, and are therefore thought to play a very important central role in T cell-mediated immune reactions. In general, it is believed that immature dendritic cells are more "tolerogenic" than mature dendritic cells, while mature dendritic cells are more "immunogenic" than their precursors. Dendritic cells are generated ex vivo from monocytes and CD34-positive cells (bone marrow cells).

In the present specification, "tumor" or "cancer" is not particularly limited, and examples thereof include breast cancer, digestive/gastrointestinal cancer, anal cancer, vermiform cancer, extrahepatic bile duct cancer, gastrointestinal carcinoid tumor, colon cancer, esophageal cancer, gallbladder cancer, gastric cancer, gastrointestinal stromal tumor (gist), islet cell carcinoma, adult primary liver cancer, pediatric liver cancer, pancreatic cancer, rectal cancer, small intestine cancer, and gastric cancer; endocrine and neuroendocrine cancers, such as pancreatic adenocarcinoma, adrenocortical carcinoma, pancreatic neuroendocrine tumor, Merkel cell carcinoma, non-small cell lung neuroendocrine tumor, small cell lung neuroendocrine tumor, parathyroid cancer, pheochromocytoma, pituitary tumor and thyroid cancer; ocular bulb cancers, such as intraocular melanoma and retinoblastoma; genitourinary cancers, such as bladder cancer, kidney (renal cell) cancer, penile cancer, prostate cancer, transitional cell renal pelvis and ureter cancer, testicular cancer, urethral cancer and Wilms' tumor; germ cell cancers, such as, pediatric central nervous system cancer, pediatric extracranial germ cell tumor, extragonadal germ cell tumor, ovarian germ cell tumor; gynaecological cancers, such as cervical cancer, endometrial cancer, gestational trophoblastic tumor, epithelial ovarian cancer, ovarian germ cell tumor, uterine sarcoma, vaginal cancer and vulvar cancer; head and neck cancers such as hypopharyngeal carcinoma, laryngeal carcinoma, lip and oral cavity carcinoma, metastatic squamous cell carcinoma of neck of unknown primary origin, oral carcinoma, nasopharyngeal carcinoma, oropharyngeal carcinoma, sinus and nasal cavity carcinoma, parathyroid carcinoma, pharyngeal carcinoma, salivary gland carcinoma, and throat carcinoma; leukemias, such as adult acute lymphocytic leukemia, pediatric acute lymphocytic leukemia, adult acute myelogenous leukemia, pediatric acute myelogenous leukemia, chronic lymphocytic leukemia, chronic myelogenous leukemia, and hairy cell leukemia; multiple myelomas, such as malignant plasma cells; lymphomas, such as AIDS-related lymphoma, cutaneous t-cell lymphoma, adult Hodgkin's lymphoma, pediatric Hodgkin's lymphoma, Hodgkin lymphoma during pregnancy, mycosis fungoides, adult non-Hodgkin lymphoma, pediatric non-Hodgkin lymphoma, non-Hodgkin lymphoma during pregnancy, primary central nervous system lymphoma, Sezary's syndrome and Waldenstrom's macroglobulinemia; musculoskeletal carcinomas, such as Ewing's sarcoma, osteosarcoma and malignant fibrous histiocytoma of bone, pediatric rhabdomyosarcoma and soft-tissue sarcoma; neurocancers, such as adult brain tumors, pediatric brain tumors, astrocytoma, brainstem glioma, central nervous system atypical teratomas/rhabdomyoid tumors, central nervous system embryoma, craniopharyngioma, ependymoma, neuroblastoma, primary central nervous system (CNS) lymphoma; respiratory/thoracic cancers, such as non-small cell lung cancer, small cell lung cancer, malignant mesothelioma, thymoma and thymic carcinoma; and skin cancers, such as Kaposi's sarcoma, melanoma and squamous cell carcinoma, carcinoma of unknown primary origin.

As used herein, "identify" or "identification" refers to visualizing the presence of neoantigens.

As used herein, "isolate" or "isolation" refers to physically separating cells of interest, such as CTCs, from other cell types present in a sample of a subject.

In the present specification, "whole exome analysis" refers to a technique of comprehensively analyzing only an exon sequence in the whole genome, and is also simply referred to as "exome analysis". Specifically, an exon region is enriched from the genome, a base sequence is determined by a next generation sequencer (NGS) or the like, the obtained read sequence is mapped to a reference sequence, then SNV is detected, and annotation is added, whereby the exon sequence can be analyzed. "Exome analysis" includes a case of analyzing only a part of exomes by a target sequence for examining only a specific region of DNA.

In the present specification, "whole genome analysis" is a technique of analyzing base sequences of all DNAs constituting the genome of a living body, and a mutation is detected from the obtained sequence information of bases. Specifically, the extracted DNA is fragmented to create a library, and the base sequence is determined (sequencing) using a next generation sequencer. Thereafter, a mutation is detected by bioinformatics analysis. In the present specification, "genome" includes not only a genome in a narrow sense (a set of chromosomes containing a minimal set of genes necessary for an organism to carry out normal life activities) but also a genome encoding genetic information not constituting a chromosome.

In the present specification, "RNA-Seq" refers to an RNA sequence or RNA sequencing, and is a technique of decoding a sequence of mRNA or miRNA in a cell for quantification of an expression level, discovery of a new transcription sequence, and the like. This is performed by analyzing read information acquired using a next-generation sequencer. In addition, the single-cell RNA-Seq is a technique of performing an RNA sequence using RNA obtained from a single cell.

In the present specification, "proteome analysis" refers to an analysis that clarifies the relationship between gene information and various proteins that complicatedly interact with each other in a cell, and is a large-scale analysis technique for the structures and functions of proteins, and can comprehensively analyze proteins. The proteome refers to the entire proteins produced in specific cells, organs, and organs. For example, the two-dimensional electrophoresis, which is a technique of separating proteins, has high resolution and can detect thousands of proteins at a time, and thus can be used as a method for separating biological samples such as proteins.

In the present specification, the "transcriptome analysis" is to analyze all mRNAs (or primary transcriptional products, transcripts) present in one or expanded cells of an organism in a similar differentiated state in a specific cell biological situation. Since mRNA is variously changed by accumulation of influences from the outside of the cell, which the cell has received in the course of development, it becomes possible to analyze the properties of the current cell in detail. Specifically, analysis is performed using a microarray or the like. For example, in a case where there are more mRNAs involved in the infinite proliferation capability, invasion, metastasis, resistance, recurrence, and the like of cancer cells, mRNAs corresponding to mutated cancer genes, mRNAs corresponding to mutated cancer suppressor genes, and mRNAs for cancer-related genes in circulating tumor cells than in cells to be compared, the properties of the circulating tumor cells can be specified.

### (Preferred embodiment)

Preferred embodiments of the present disclosure will be described below. The embodiments which will be provided below are provided for a better understanding of the present disclosure, and the scope of the present disclosure should not be limited by the following descriptions. Therefore, it is apparent that those skilled in the art can appropriately make modifications within the scope of the present disclosure in view of the descriptions in the present specification. In addition, the following embodiments of the present disclosure can be used alone or in combination.

There is provided a method for preparing a sample for identifying a neoantigen possessed by a subject by sequencing without nucleic acid amplification and/or proliferation by culture, the method including the steps of: isolating monocytes from peripheral blood mononuclear cells collected from the subject using apheresis, wherein apheresis condition for performing the apheresis includes circulating tumor cell enrichment condition; isolating circulating tumor cells from the peripheral blood mononuclear cells from which the monocytes have been isolated; optionally, confirming a purity of circulating tumor cells required for the identification of the neoantigen; and providing the circulating tumor cells as a sample for identifying the neoantigen and/or extracting DNA and/or RNA from the circulating tumor cells. The method of the present disclosure is advantageous in that, since culture propagation are not performed, amplification errors due to nucleic acid amplification and mutation introduction in culture propagation do not occur, and the diversity of malignancies in a sample derived from a subject can be reflected as it is.

According to another aspect of the present disclosure, there is provided a method for preparing a sample for identifying a neoantigen possessed by a subject by sequencing without nucleic acid amplification and/or proliferation by culture, the method including the steps of: isolating monocytes from peripheral blood mononuclear cells collected from the subject using apheresis; isolating circulating tumor cells from the peripheral blood mononuclear cells from which the monocytes have been isolated; optionally, confirming a purity of circulating tumor cells required for the identification of the neoantigen; and providing the circulating tumor cells as a sample for identifying the neoantigen and/or extracting DNA and/or RNA from the circulating tumor cells.

According to still another aspect, there is provided a method for identifying a neoantigen possessed by a subject by sequencing without nucleic acid amplification and/or expansion by culture, the method including: isolating monocytes from peripheral blood mononuclear cells collected from the subject using apheresis, wherein apheresis condition for performing the apheresis includes circulating tumor cell enrichment condition; isolating circulating tumor cells from the peripheral blood mononuclear cells from which the monocytes have been isolated; extracting DNA and/or RNA from the circulating tumor cell to create a DNA/RNA sequence library; and identifying a neoantigen based on mutation information of the sequence library, wherein the circulating tumor cells have a purity of at least about 20%.

For neoantigen identification, about 30 mg of tumor tissue is usually required by biopsy or in a surgical operation process. However, a biopsy or a surgical operation on a patient is burdensome, and a biopsy or a surgical operation only for neoantigen identification is generally not realistic. Therefore, in one embodiment of the present disclosure, a sample for identifying neoantigens can be prepared using less tumor tissue, preferably peripheral blood mononuclear cells collected from a subject using apheresis. In one embodiment, the apheresis condition can include subjecting a spillover volume and/or a buffy coat volume to the circulating tumor cell enrichment condition. In one embodiment, the apheresis condition can include a spillover volume of about 9 to about 12 ml and/or a buffy coat volume of about 7 to about 10 ml. In one embodiment, the apheresis condition other than the spillover volume and the buffy coat volume may be set in any manner as long as they are the circulating tumor cell enrichment condition, and default condition of the device can be used.

In one embodiment, apheresis can be performed using, for example, a centrifugal blood component separation device (COMTEC), and blood can be separated into blood components such as platelets, lymphocytes, white blood cells, and plasma, or plasma exchange can be performed. In one embodiment, such a device can be used to collect peripheral blood mononuclear cells. In the centrifugal blood component separation device (COMTEC), whole blood is continuously collected from a patient/blood donor, anticoagulation treatment and defoaming are performed, and then the blood is separated into main blood components using a centrifugal force. The degree of separation is determined by the position of an interface, the blood flow and the speed of centrifugation. In addition, the blood components are separated from the outside in the order of red blood cells-granulocytes-(monocytes, lymphocytes, stem cells)-platelets by size or specific gravity in a centrifuge, and plasma is separated to the innermost side. A separation chamber has outlets for various blood components, and has a mechanism in which components other than the blood components to be collected are mixed again in a drip chamber and returned to the patient/blood donor.

In order to position the blood cells in the separation chamber of this device, the chamber has a row of holes through which light passes, and a boundary of an interface (boundary between an outer cell fraction and an inner plasma fraction) is automatically detected by an optical sensor by means of the row of holes.

There is a light source in a lower part of the centrifuge, and light is projected from the lamp toward a row of holes, and an interface receiver (optical sensor) detects the position of a boundary (interface) between opaque cells and transparent plasma from light passing through the holes. In a portion where plasma is present, light passes through holes therein, and thus the holes are counted. Light does not pass through holes in the presence of blood cells, and thus the holes are not counted. The degree of blood separation can be confirmed by the number of holes counted. That is, in a case where the position of the blood cells in the chamber (how many holes are blocked by the cells) is set in advance by the device, if the number of holes is small as compared with that at the actual position of the separation interface, the plasma is accumulated in the chamber, and a plasma pump is stopped so that the interface can be pushed down.

In one embodiment, the accumulated blood components of interest are accumulated in a storage bag by a collecting pump via an outlet port for the plasma through a step of raising the position of the interface, called spillover.

The specification of the centrifugal blood component separation device (COMTEC) will be shown below.
1. Centrifuge
   Inner rotor rotation speed: 300 to 2200 rpm
   Outer rotor rotation speed: 150 to 1100 rpm
   Maximum rotation speed: 2200 rpm
2. Pump
   Form: line roller pump
   Speed range: 0.3 to 21 rpm (collection pump, plasma pump, whole blood pump, ACD pump/circulation pump)
   1 to 28 rpm (ACD pump)
   Flow rate range: blood collection: 12.4 to 120 mL/min
   plasma: 5.12 to 80 mL/min
   collection: 1.78 to 80 mL/min
   anticoagulant: 1.78 to 17.5 mL/min
3. Program type
   Plt-5d: collection of platelets for up to 5 days of storage by double needle method
   Plt-5d-SN: collection of platelets for up to 5 days of storage by single needle method
   PBSC Lymphocyte: separation of peripheral blood stem cells and lymphocytes
   RV-PBSC: collection of enriched peripheral blood stem cells enriched in small amount
   BMSC: separation of stem cells from bone marrow
   Granulocyte: collection of granulocytes
   MNC: collection of mononuclear cells
   Removal: therapeutic removal of plasma or plasma and lymphocytes
   TPE: therapeutic plasma exchange
   Adsorption: therapeutic plasma separation
   RBC: therapeutic red blood cell exchange and removal

When peripheral blood mononuclear cells are collected using the centrifugal blood component separation device as described above, data such as sex, height, body weight, hematocrit value, and number of white blood cells of the patient can be set. A circulating blood volume of the patient is grasped by the sex, height, and body weight, and an appropriate blood collection flow rate is set on the basis of the data. In addition, a value of the plasma pump can be determined by setting the hematocrit value. From the number of white blood cells, it is possible to calculate a numerical value for the purpose of determining how much blood should be processed to form an appropriate buffy coat and to efficiently collect the blood. In one embodiment, the numerical value "spillover volume" for efficiently recovering cells in the middle of blood collection and the "buffy coat volume" for determining how much blood is collected can also be set.

Hereinafter, steps of blood component separation in the case of using COMTEC will be described.

First, the device is powered on and a dedicated disposable blood circuit (apheresis set) is attached. After the separation chamber is attached to a chamber holder, priming is performed. In one embodiment, the blood components can be separated as follows.
(1) Completely degas a saline line and make preparations before separation according to a display.
(2) Press a (Continue) key to puncture the blood donor with a blood sampling needle and collect a pre-blood donation sample. Puncture the blood donor with a retransfusion needle.
(3) Input patient information.
(4) Press a (Start) key to start separation. The separation and the blood collection are automatically performed, and automatically terminated when the target setting value is reached.

In one embodiment, when COMTEC is used, retransfusion can be performed as follows after separation of the blood components.
(1) Press the (Continue) key to open a clamp of the saline line, remove a blood collection line from the blood donor, and make preparations before retransfusion (in the case of the single needle method, the blood collection line is not removed).
(2) Press the (Start) key to start retransfusion. The retransfusion is automatically performed and ends.
(3) After completion of retransfusion, press a (Stop) key.

In one embodiment, condition for the spillover can be appropriately set. The spillover volume greatly affects the collection efficiency of cells, and thus is regulated so that cells can be efficiently recovered. For example, it can be varied depending on the condition of the blood of the subject, and can be adjusted depending on the presence or absence of numbness in the subject, the physical condition thereof, and the like. In one embodiment, the spillover volume can be a value to achieve circulating the circulating tumor cell enrichment condition, e.g., the spillover volume can be about 5 to about 15 ml, preferably about 7 to 14 ml, and more preferably about 9 to about 12 ml.

In one embodiment, the buffy coat volume can be appropriately set so that the cells of interest can be collected. In one embodiment, the buffy coat volume can be a value to achieve circulating the circulating tumor cell enrichment condition, e.g., the buffy coat volume can be about 5 to about 15 ml, preferably about 6 to 12 ml, and more preferably about 7 to about 10 ml.

In one embodiment, to achieve the circulating tumor cell enrichment condition, the spillover volume and buffy coat volume can also be used in combination of the above condition.

In one embodiment, the centrifugal blood component separation device is not limited to the above specific device, and may be any device as long as it can collect peripheral blood mononuclear cells. As such a centrifugal blood component separation device, Spectra Optia (Terumo Corporation) or the like can be used in addition to COMTEC.

Hereinafter, steps of blood component separation in the case of using Spectra Optia will be described.

First, the device is powered on, a blood circuit is attached to the device, and the circuit is tested and primed. Thereafter, the patient/donor is connected according to the instructions on a screen, clamps of a blood collection line and a retransfusion line are opened, and the blood components are separated.

In one embodiment, since information is displayed on the screen during the separation of the blood components, the patient/donor status can always be monitored, and the concentrations at a collection port, an RBC interface, the collection line can be monitored. When an alarm is displayed or when it is desired to change a condition or the like during processing, adjustment can be performed according to instructions on the screen, and the operation can be restarted.

In one embodiment, in the case of using Spectra Optia, the following component separation steps can be continuously performed.
(1) The patient/donor's whole blood introduced into the blood collection line attached to the circuit is introduced into a channel portion in the circuit attached to a centrifugal separator by the action of a blood collection pump.
(2) An anticoagulant is used at a certain ratio so that the blood does not coagulate. The anticoagulated blood is returned to the patient/donor.
(3) As the centrifugal separator rotates, the blood in the channel is separated for each component.
(4) Each component after centrifugation is divided into three lines of tubes for plasma, platelets, and red blood cells, flows out from the centrifugal separator, and enters a cassette section in the circuit by a pump operation. Thereafter, the blood is introduced into either a collection bag or a retransfusion reservoir configured in the cassette section through a branch section by a valve operation.
(5) The blood guided to the retransfusion reservoir is returned to the patient/donor via the retransfusion line by the action of a retransfusion pump. In an Exchange set, a replacement liquid is introduced into the reservoir in the cassette through a replacement liquid line by a replacement liquid/collection pump and supplied to the patient.

In one embodiment, each step described above can be monitored by each safety function shown in the safety device, and can be designed so that each function works according to a situation when an event related to the safety function occurs. Each operation can be performed on a touch screen monitor, and patient/donor information and an operation program necessary for the operation can be input to an automatic control computer through this screen. In addition, communications with an external information device can be performed from an Ethernet port, but no electronic device is connected to the Ethernet in a patient/donor environment.

In one embodiment of the present disclosure, the number of peripheral blood mononuclear cells isolated by apheresis may be an amount sufficient to isolate monocytes or circulating tumor cells, and can be, for example, at least about 10 or more, about 1 × 10² or more, about 1 × 10³ or more, about 1 × 10⁴ or more, about 1 × 10⁵ or more, about 1 × 10⁶ or more, about 1 × 10⁷ or more, or about 1 × 10⁸ or more.

In one embodiment, the sample subjected to apheresis can be any sample suitable for identifying neoantigens, and can include, for example, whole blood, bone marrow, pleural fluid, ascites, central spinal fluid, urine, saliva, and bronchial lavage. In one embodiment, the sample is blood, such as whole blood or any fraction or component thereof. A blood sample suitable for use in the present disclosure can be extracted from any source known to contain blood cells or components thereof, such as arteries, veins, peripheral tissues, umbilical cords, or the like. For example, the sample subjected to apheresis is acquired and processed using well-known and conventional clinical methods (e.g., procedures for aspirating and processing whole blood). In one aspect, an exemplary method can be aspiration of peripheral blood from a cancer patient.

The PBMCs obtained by apheresis mainly consist of T lymphocytes and monocytes. Isolation of monocytes from the PBMCs can be performed using any technique known in the art. For example, in one embodiment of the present disclosure, the PBMCs are separated and seeded, and a supernatant containing floating cells in which circulating tumor cells and lymphocytes are present is recovered, whereby monocytes can be isolated.

In one embodiment of the present disclosure, the circulating tumor cells can be isolated from the peripheral blood mononuclear cells from which monocytes have been isolated. The isolation of circulating tumor cells from PBMCs can be performed using any technique known in the art. For example, in one embodiment of the present disclosure, CTCs can be isolated from a fraction collected as the supernatant containing floating cells by a technique such as isolation with a monoclonal antibody, size fractionation, or fractionation based on specific gravity.

In one embodiment, the monoclonal antibody used in isolation of CTCs from peripheral blood mononuclear cells from which the monocytes have been isolated can include, for example, an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody. In one embodiment, an appropriate antibody can be appropriately selected depending on the type of cancer. For example, in the case of EpCAM-negative pancreatic cancer, CTCs can be isolated by CD 45- Vimentin+ or CD45- N-Cadherin+.

In another embodiment, the isolation of CTCs from peripheral blood mononuclear cells from which monocytes have been isolated can be performed by size fractionation using a cell search system, a metal mesh, or the like, and, for example, a filter kit for CTC recovery (ScreeCell, Inc.) or a metal mesh (Murata Manufacturing Co., Ltd.) can be used. In one embodiment, when a filter kit for CTC recovery (ScreeCell, Inc.) is used, CTCs can be collected on a filter in the process of passing whole blood through the filter. Since no antibody is used, EMT (epithelial-mesenchymal transition)-transformed cells with little expression of EpCAM (epithelial cell adhesion molecule) can also be collected. In another embodiment, when a metal mesh (Murata Manufacturing Co., Ltd.) is used, cells to be captured and cells to be passed can be selected by selecting an appropriate mesh size, and thus only CTCs can be captured by selecting a mesh size suitable for the size of target CTCs.

In one embodiment, the isolation of CTCs from peripheral blood mononuclear cells from which monocytes have been isolated can also be performed by fractionation based on the specific gravity using an elutriator or the like. Since the blood components have different densities, density centrifugation (for example, Ficoll-Paque) (CYTIVA, Marlborough, MA, USA) can also be used to separate peripheral blood mononuclear cells and CTCs from whole blood. In other embodiments, a system in which a density gradient centrifugation system and a porous barrier that increases depletion of mononuclear cells are combined, or a gradient centrifugation method is incorporated with an immunoaffinity approach, and unnecessary PBMC cells are crosslinked to red blood cells in a sample to form an immunorosette, whereby the separation efficiency of CTCs can be greatly improved.

In one embodiment, when monoclonal antibodies are used to isolate CTCs, a cell sorter can be used to sort the CTCs and control cells (antibody-positive cells). For example, a cell sorter SH800 (Sony Corporation) can be used, and an example of an operation procedure thereof is as follows.

### Power ON

- PC monitor, keyboard, mouse
- Compressor
- Main body
- PC

*Check whether water is in the tank of the main body.

### <PC Screen>

```
          PW: fcm
           Start desktop software
           ↓
          Register chip (100 µm)
           ↓
          NEXT → PUSH OPEN
           ↓
           Open the door of the chip, discard old chip if any
           ↓
           Insert the chip into CHIP INSERT (orientation in which characters face front)
           ↓
          NEXT
           ↓
          Laser setting, filter setting
           ↓
          NEXT
           ↓
          Wait a little while in this state. When setting is completed, the status
          becomes "successfully". → Calibration (put beads in 5 ml tube)
           ↓
           Set the beads on the main body and start (standard, not place a check mark in
 □) ↓
          Wait a little while in this state. When calibration is completed, the status
          becomes "successfully".
           ↓
           OK
           ↓
           Open template (CD45, EpCAM template)
           ↓
           Create new experiment
           ↓
          Flow a little. Flow a little at about Sample pressure 5 and stop.
           ↓
           Check the screen of flow, apply gate, and set the place for cell collection.
           ↓
           Input sections where cells are desired to be collected and the number thereof
 in each of L and R
          Method: 2Way, Mode: Normal
           ↓
           Set tubes into which the collected cells are placed and close the door (place
 about 6 ml of CML in a 15 ml tube).
           ↓
          Load Collection
           ↓
           The tubes are set at the back.
           ↓
          Experiment file
           ↓
           Change name
           ↓
           Set sample to be flowed
           ↓
           Start
           ↓
           Once stable, start sort. At that time, record is also started
           ↓
           The sort stops for the tube in which the desired number of cells to be collected
 is reached. Stop when less sample remains.
```

In one embodiment of the present disclosure, sorting can be performed in the following procedure.

```
Dissolve frozen cells at 37°C
           ↓
          Filter: Falcon remove aggregated cells
           ↓
           3 ml PBS Buffer (2 mM EDTA, 2% FBS)
           ↓
           Centrifuge at 1000 rpm, for 2 min, and at 4°C
           ↓
           Count cells→Centrifuge at 1000 rpm, for 2 min, and at 4°C
           ↓
           Stain with antibodies
           Antibody concentration
           CD45 PE 5 µg/ml
          EpCAM 10 µg/ml
           ↓
          Place on ice for 30 min
           ↓
           Add 2 ml PBS Buffer (2 mM EDTA, 2% FBS)
           ↓
           Centrifuge at 1000 rpm, for 2 min, and at 4°C
           ↓
           Suspend in 1 ml PBS Buffer (2 mM EDTA, 2% FBS)
           ↓
          Filter, tube
           ↓
           CML 6 ml
           CML
           1% MEM Non-Essential Amino Acis Solution 1% HEPES
           1% Sodium Pyruvate
           50 µM 2-mercaptoethanol
           1% Penicillin-Streptomycin
           10% FCS (FBS)
           500 ml RPMI 1640 medium
           ↓
           Sorting (CD45 PE, EpCAM FITC)
           ↓
           Centrifuge at 1800 rpm, for 10 min, and at 4°C
           ↓
           Suspension RLT Buffer 350 µl
          RLT Buffer:
          Buffer RLT Plus (QIAGEN) 1 ml
           β-ME 10 µl
```

In one embodiment of the present disclosure, the purity of CTCs isolated by monoclonal antibodies or size fractionation may be a purity necessary for identification of neoantigens, or a purity sufficient to extract DNA or RNA, and can be, for example, at least about 10%, about 20%, about 30%, about 40%, about 50%, about 60%, about 70%, about 80%, about 85%, about 90%, about 95%, about 97%, or the like.

In one embodiment of the present disclosure, the number of CTCs isolated by monoclonal antibodies or size fractionation may be an amount necessary for identification of neoantigens or an amount sufficient to extract DNA or RNA, and can be, for example, at least about 10 or more, about 1 × 10² or more, about 1 × 10³ or more, about 1 × 10⁴ or more, or about 1 × 10⁵ or more.

In one embodiment of the present disclosure, the circulating tumor cells isolated from the peripheral blood mononuclear cells can be frozen. In this case, the circulating tumor cells can be cryopreserved at a temperature or for a time which does not affect the quality thereof that can be provided as a sample for identifying neoantigens or the quality thereof that allows for extraction of DNA and/or RNA from the circulating tumor cells, and, for example, can be cryopreserved for about 1 year or more in liquid nitrogen (-196°C) or an ultra-low temperature tank (-80°C).

In one embodiment of the present disclosure, DNA and/or RNA can be extracted from the circulating tumor cells isolated from the peripheral blood mononuclear cells. An amount of DNA or RNA to be extracted may be any amount that can be used in DNA/RNA sequencing, and, for example, at least about 100 pg of DNA and/or RNA can be extracted. In other embodiments, the amount of DNA and/or RNA to be extracted can be at least about 200 pg, about 500 pg, about 800 pg, about 1 ng, about 10 ng, about 30 ng, about 50 ng, about 80 ng, about 100 ng, or about 200 ng.

In one embodiment of the present disclosure, DNA extraction can be performed in the following procedure.

```
          DNA extraction with QIamp (registered trademark) DNA Mini
           20 µl Proteinase K ← 1.5 ml microtube
           ↓
           Add the sample suspended in 200 µl PBS to this tube
           ↓
           Add 200 µl Buffer AL and sufficiently mix by vortexing
           ↓
           Incubate at 56°C for 10 min
           ↓
           Spin down to recover solution attached to inside of the lid
           ↓
           Add 200 µl of 100% ethanol and sufficiently mix by vortexing
           ↓
           Add the sample to QIamp Mini Spin Column
           ↓
           Close the lid and centrifuge at 6,000 g for 1 min
           ↓
          Discard filtrate and collection tube and attach new collection tube
           ↓
           Add 500 µl Buffer AW1
           ↓
           Close the lid and centrifuge at 6,000 g for 1 min
           ↓
           Add 500 µl Buffer AW2
           ↓
           Close the lid and centrifuge at 12,000 g for 3 min
           ↓
          Discard filtrate and centrifuge at 12,000 g for 1 min
           ↓
           Transfer QIamp Mini Spin Column to new 1.5 ml microtube
           Add 100 µl Buffer AE and incubate at room temperature for 1 min
           ↓
           Centrifuge at 6,000 g for 1 min
          Measure O.D and store extracted DNA at -80°C
```

In other embodiments of the present disclosure, DNA extraction can be performed in the following procedure.

```
          Extract DNA with NucleoSpin (registered trademark) Blood L (TAKARA)
           Add Proteinase K: Proteinase Buffer PB and dissolve (store at -20°C)
           Add Wash Buffer B5: 100% ethanol
           ↓
           Sample (up to 2 × 107 cells) cells (pellet-shaped) are used
           ↓
          Dissolve in 2 ml PBS
           ↓
           Charge 150 µl Proteinase K
           ↓
           2 ml BQ1 vigorously vortex (10 sec)
           ↓
           Incubation at 56°C for 15 min ← Heat block is warmed in advance
           ↓
           Add 2 ml of 100% ethanol and mix
           ↓
          DNA binding
          Load 3 ml NucleoSpin Blood L Column into the sample for the first time, and
 centrifuge at 4,500 g, for 3 minutes, and at 25°C
           ↓
          Load 3 ml NucleoSpin Blood L Column into the sample for the second time,
 and centrifuge at 4,500 g, for 5 minutes, and at 25°C
           ↓
          First washing
           2 ml BQ2, centrifuge at 4,500 g, for 2 min, and at 25°C
           ↓
           Second washing, Dry membrane
           2 ml BQ2, centrifuge at 4,500 g, for 10 min, and at 25°C
           ↓
          DNA extraction, new 15 ml tube ← Allow Elution Buffer EB to warm to
 70°C
           200 µl Buffer BE (preheated to 70°C) 2 min, incubation at room temperature
           Centrifuge at 4,500 g, for 2 min, and at 25°C
           ↓
           Store the extracted DNA at -80°C
```

In one embodiment, the extraction of RNA can be performed using a kit or technique known in the art, and, for example, Rneasy mini kit (QIAGEN) can be used. In the case of using the Rneasy mini kit (QIAGEN), RNA can be extracted by the following procedure.

```
Add to gDNA Eliminator spin column
           ↓
           Centrifuge at 10,000 g, for 1 min, and at 25°C
           ↓
           Add 350 µl of 70% ethanol to the liquid dropped by centrifugation
           ↓
           Add the whole amount of the liquid to Rneasy Min Elute spin column
           ↓
           Centrifuge at 10,000 g, for 1 min, and at 25°C
           ↓
          Buffer RW1 700 µl
           ↓
           Centrifuge at 10,000 g, for 1 min, and at 25°C
           ↓
          Buffer RPE 500 µl
           ↓
           Centrifuge at 10,000 g, for 1 min, and at 25°C
           ↓
           500 µl of 80% Ethanol
           ↓
           Centrifuge at 10,000 g, for 2 min, and at 25°C
           ↓
          Dry, open the lid, and centrifuge at 12000 g, for 5 minutes, and at 25°C
           ↓
          Rnase-free water 14 µl
           ↓
           Centrifuge at 12,000 g, for 1 min, and at 25°C
           ↓
           Quantify (NANO DROP ONE, Thermo SCIENTIFIC)
```

In other embodiments, when RNA is extracted using kiazole and triazole, the following procedure can be employed.

```
          Dissolve frozen cells
           ↓
           Suspend in 3 ml PBS (2% FBS, 2 mM EDTA)
           ↓
           Centrifuge at 1000 rpm, for 2 min, and at 4°C
           ↓
           Cell number measurement, 1.91 × 106 cells
           ↓
           Separate the cells into 2 tubes (500 µl each) (15 ml tube)
           ↓
           Centrifuge at 1000 rpm, for 2 min, and at 4°C
           ↓
           QIAzol Lysis reagent 700 µl
           TRIzol Reagent 700 µl
           Suspend in each
           ↓
           Chloroform 140 µl
           ↓
           Vortex, stir well until it turns white
           ↓
           Centrifuge at 12,000 g, for 15 min, and at 4°C
           ↓
           Take only water layer (300 µl)
           ↓
           100% Ethanol, 1.5 times volume the taken water layer (450 µl)
           ↓
           Add to column (in 2 portions)
           ↓
           Centrifuge at 8000 g, for 1 min, and at 25°C
           ↓
          Buffer RWT 700 µl
           ↓
           Centrifuge at 8000 g, for 1 min, and at 25°C
           ↓
          Buffer RPE 500 µl
           ↓
           Centrifuge at 8000 g, for 1 min, and at 25°C
           ↓
           500 µl of 80% Ethanol
           ↓
           Centrifuge at 8000 g, for 2 min, and at 25°C
           ↓
           Centrifuge 12000 g, for 5 min, and at 25°C
           ↓
          Elution, Rnase free Buffer 14 µl (elution with CTC 20 001 → 20 µl)
           ↓
           Allow to stand at room temperature for 2 to 3 min
           ↓
           Centrifuge at 12,000 g, for 1 min, and at 25°C
```

According to one aspect of the present disclosure, there is provided a method for producing a cell for dendritic cell therapy using a neoantigen possessed by a subject, the method including: isolating monocytes from peripheral blood mononuclear cells collected from the subject using apheresis, wherein apheresis condition for performing the apheresis includes circulating tumor cell enrichment condition; isolating circulating tumor cells from the peripheral blood mononuclear cells from which the monocytes have been isolated; extracting DNA and/or RNA from the circulating tumor cell to create a DNA/RNA sequence library; identifying a neoantigen based on mutation information of the sequence library; and introducing the neoantigen into dendritic cells.

In one embodiment of the present disclosure, the dendritic cells may be those obtained from the subject by apheresis, or those induced from monocytes isolated from peripheral blood mononuclear cells collected from the subject can also be utilized.

In one embodiment, in the production of dendritic cells from a frozen raw material, the dendritic cells for dendritic cell therapy can be obtained by performing cryopreservation/conveyance of the apheresis raw material, raw material thawing/washing/centrifugation, removal of supernatant and floating cells after adhesion treatment, dendritic cell induction, dendritic cell maturation/activation, and the like.

In one embodiment of the present disclosure, it is possible to isolate monocytes from peripheral blood mononuclear cells collected from the subject using apheresis, to collect CTCs from PBMCs after removal of the monocytes, and to use the CTCs to identify a neoantigen protein expressed in the CTCs can be identified by proteome analysis of the CTCs.

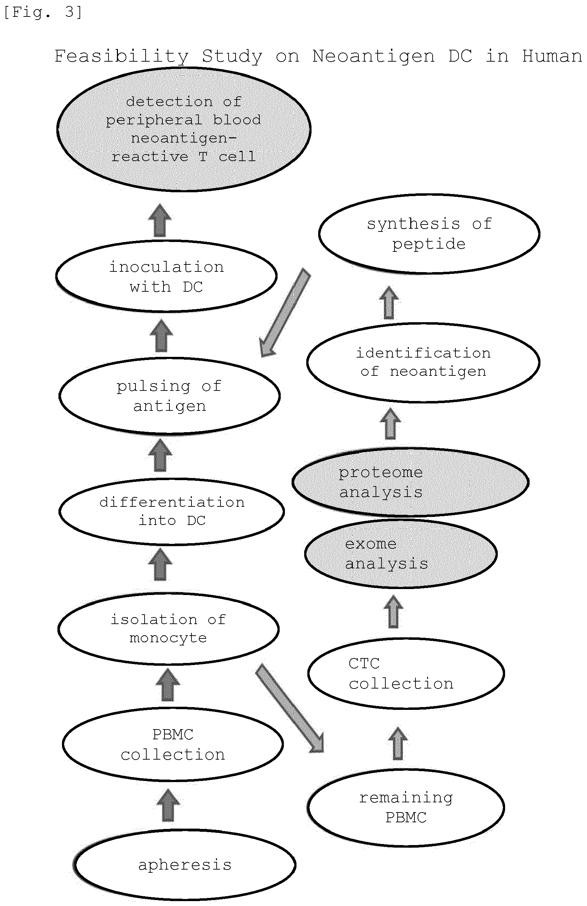

In this case, for the neoantigen identification, it is possible to extract DNA from CTCs and normal cells, to perform exome analysis, and then to identify the neoantigen by software. The neoantigen protein expressed in the CTCs can be identified by proteome analysis of the CTCs, and repertoire analysis and Elispot analysis can be used for analysis of peripheral blood T cells after administration of DCs. Thus, the peptide of the neoantigen identified using the CTCs is pulsed to the DCs to confirm whether or not neoantigen-reactive T cells can be detected in the peripheral blood of a cancer patient (FIG. 3).

The proteome analysis can be analyzed by any technique, and is not particularly limited as long as it can be analyzed using CTCs, but, for example, the proteome analysis can be performed by the following method.
(1) A suspension containing about 1000 to tens of thousands of CTCs is centrifuged at 1000 to 2000 cpm for 10 to 15 minutes to make cell pellets. The cell pellets are stored in a low temperature tank at -80°C or a liquid nitrogen container.
(2) Proteins are extracted from the pellets of the CTCs by any method of surfactant, freezing-thawing, and osmotic shock.
(3) The extracted proteins are analyzed by an apparatus (liquid chromatograph mass spectrometer "Liquid Chromatograph-Mass Spectrometry: LC-MS/MS") in which a high performance liquid chromatograph (HPLC) and a triple quadrupole mass spectrometer (MS/MS) are combined. Thus, the proteins expressed in the CTCs are identified.

In one embodiment of the present disclosure, exome analysis can be performed using CTCs isolated as described above. The exome analysis can be analyzed by any technique, and is not particularly limited as long as it can be analyzed using CTCs, but, for example, the proteome analysis can be performed by the following method.
(1) A library for whole exome sequence analysis is created from total DNA of CTCs and control cells using SureSelect All Exon kit from Agilent technologies. Quality inspection of the created sequence library is performed using TapeStation from Agilent.
(2) Sequencing is then performed by NovaSeq 6000 from Illumina, Inc.
(3) Reads obtained by sequencing are mapped using GEM (Marco-Sola, et al., 2012). Mapping can be performed by Subread (Liao, et al., 2013), HISAT/HISAT2 (Kim, et al., 2015), or KART (Lin and Hsu, 2017). Next, the number of reads mapped by HTSeq is counted, and gene expression analysis is performed. From map results, variant call is performed using The Genome Analysis Toolkit (GATK) "HaplotypeCaller" (Version: 4.0.10.1) to create a vcf file.

In one embodiment of the present disclosure, the CTCs obtained as described above can be used for various analyses other than the neoantigen identification. Thus, according to another aspect of the present disclosure, there is provided a method for preparing a sample containing circulating tumor cells derived from a subject for analysis without nucleic acid amplification and/or proliferation by culture, the method including the steps of: isolating monocytes from peripheral blood mononuclear cells collected from the subject using apheresis, wherein apheresis condition for performing the apheresis includes circulating tumor cell enrichment condition; isolating circulating tumor cells from the peripheral blood mononuclear cells from which the monocytes have been isolated; optionally, confirming a purity of circulating tumor cells required for the analysis; and optionally, performing pretreatment for the analysis on the circulating tumor cells.

According to still another aspect of the present disclosure, there is provided a method for analyzing circulating tumor cells derived from a subject without nucleic acid amplification and/or proliferation by culture, the method including the steps of: isolating monocytes from peripheral blood mononuclear cells collected from the subject using apheresis, wherein apheresis condition for performing the apheresis includes circulating tumor cell enrichment condition; isolating circulating tumor cells from the peripheral blood mononuclear cells from which the monocytes have been isolated; and performing analysis using the circulating tumor cells.

In one embodiment, such analysis is not particularly limited as long as it can be performed using CTCs, and can include, for example, whole exome analysis, whole genome analysis, RNA-Seq, single cell RNA-Seq, proteome analysis, and transcriptome analysis. In one embodiment, the sample containing circulating tumor cells prepared by the method of the present disclosure can be used for identifying the neoantigen possessed by the subject by performing the analysis as described above. In one embodiment, the features described in other parts of the present specification can be appropriately adopted even when it is used for other analyses.

In one embodiment of the present disclosure, the circulating tumor cells can be appropriately pretreated according to the analysis of interest, and DNA, RNA, or protein can also be extracted from the circulating tumor cells. Such extraction means is not particularly limited, and a known extraction technique can be used as long as it can be extracted in an amount or with accuracy that can be used for the analysis of interest.

The amount of DNA or RNA to be extracted may be any amount that can be used in the subsequent analysis, and, for example, at least about 100 pg of DNA and/or RNA can be extracted. In other embodiments, the amount of DNA and/or RNA to be extracted can be at least about 200 pg, about 500 pg, about 800 pg, about 1 ng, about 10 ng, about 30 ng, about 50 ng, about 80 ng, about 100 ng, or about 200 ng.

In one embodiment, such DNA or RNA can be used to create a DNA/RNA sequence library. For example, in the case of identifying a neoantigen, the neoantigen can be identified based on mutation information of this sequence library.

In one embodiment, as described above, it is also possible to identify the neoantigen protein by extracting CTCs and DNA derived from normal cells, then performing exome analysis, and performing proteome analysis of some of the CTCs.

According to another aspect of the present disclosure, there is provided a method for treating or preventing a subject by analyzing circulating tumor cells without nucleic acid amplification and/or proliferation by culture, the method including the steps of: isolating monocytes from peripheral blood mononuclear cells collected from the subject using apheresis, wherein apheresis condition for performing the apheresis includes circulating tumor cell enrichment condition; isolating circulating tumor cells from the peripheral blood mononuclear cells from which the monocytes have been isolated; performing analysis using the circulating tumor cells; and treating or preventing the subject based on results of the analysis.

In one embodiment, the features described in other parts of the present specification can be appropriately adopted in such a treatment or prevention method.

In the present specification, "or" is used when "at least one or more" of items listed in a text can be employed. The same applies to "or". When "two values" "within a range" are explicitly stated in the present specification, the range includes the two values themselves.

References cited herein, such as scientific literature, patents, and patent applications, are incorporated herein by reference in their entirety to the same extent as each is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described herein, but is limited only by the claims.

### Examples

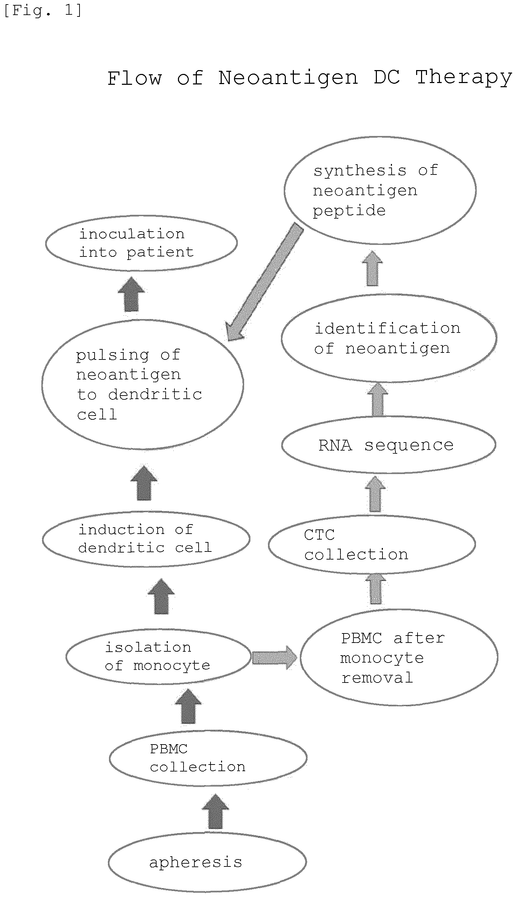

Hereinafter, the procedure for making neoantigen dendritic cells will be described (FIG. 1).

### (Example 1: Collection of peripheral blood mononuclear cell (PBMC) fraction by apheresis)

Examples of the centrifugal blood component separation device for apheresis include COMTEC (COM.TEC) (registered trademark) (Fresenius Kabi) and Spectra Optia (Terumo Corporation).

Large veins near the cubital fossae of the right hand and the left hand (if not in this place, search for other places) are punctured with 18 G needles one by one, and one is secured as a blood collection route and the other is secured as a blood retransfusion route. Both routes are attached to the centrifugal blood component separation device, and PBMCs are collected from a subject (patient). For the program of the centrifugal blood component separation device, "MNC: collection of mononuclear cell" is selected. Over about 1 to 3 hours, 1 × 10⁸ to 1 × 10⁹ or more PBMCs are collected. The PBMCs include monocytes, lymphocytes, circulating cancer cells (CTCs), and the like, and hardly include red blood cells, granulocytes, and platelets. The PBMCs are stored together with some plasma in a storage bag attached to the centrifugal blood component separation device.

It is shown here that the apheresis condition is preferably a spillover volume of about 9 to about 12 ml and/or a buffy coat volume of about 7 to about 10 ml. In this example, the spillover volume was adjusted to 10 to 11 ml, the buffy coat volume was set to 8 ml, and the other condition was set to default settings for mononuclear cell collection in the device.

### (Example 2: Isolation of monocyte forming dendritic cell from PBMC fraction)

### (1) Separation and seeding of PBMC

In a plurality of 50 ml centrifuge tubes, 20 ml of Ficoll is placed. A solution containing PBMCs (20 to 25 mL) in the storage bag is overlaid on Ficoll in each of the centrifuge tubes. Centrifugation is performed at 20°C and 500 G for 30 minutes, and a buffy coat containing PBMCs is collected with a pipette, and placed in the 50 ml centrifuge tube. PBS is added, and centrifugation and supernatant removal are repeated 3 to 5 times. Finally, an AIM medium is added to attain 40 ml. A small amount of the AIM medium in which PBMCs are suspended is taken, the number of cells is calculated, and a blood image test is performed to measure the proportion of monocytes. The total number of monocytes obtained is determined from these numerical values.

A required amount of AIM medium is added to the AIM medium in which PBMCs are suspended so that the final number of monocytes is 1 to 2 × 10⁷/6 ml in one petri dish. Each 6 ml portion of the PBMC-containing AIM medium adjusted is seeded in each petri dish and placed in an incubator for 30 minutes. After confirmation of attachment of the cells to the bottom surface with a microscope, the supernatant is removed. The supernatant containing floating cells is recovered in a centrifuge tube. After addition of 6 ml of fresh AIM medium, the medium is placed in the incubator and cultured for 16 to 24 hours.

### (2) Storage of cell fraction containing CTC

(1) CTCs and lymphocytes are present in the supernatant containing floating cells obtained in (1). The number of cells in the suspension and the survival rate of the cells are confirmed. The suspension is dispensed into a centrifuge tube and then centrifuged to remove the supernatant. To 1 to 3 × 10⁷ cells, 1 ml of Cell Banker (registered trademark) is added and pipetted. The cell suspension is dispensed into a cryotube and cryopreserved in a deep freezer at -80°C until transport to a facility where the CTCs will be isolated.

### (3) Induction of differentiation of monocyte

The petri dish is taken out from the incubator, and the culture surface thereof is washed using a culture supernatant. After the washing, the washing liquid is removed, followed by washing with 10 mL of AIM medium or PBS to remove the supernatant. An AIM medium containing IL-4 at a final concentration of 50 ng/ml and GM-CSF at a final concentration of 50 ng/ml is added in an amount of 6 to 8 mL per petri dish. Thereafter, the medium is placed in the incubator and cultured for 5 days for induction of immature dendritic cells.

After the culturing, the petri dish is taken out from the incubator. The culture supernatant is removed and 2 ml of AIM medium is added. The cells stuck to the bottom surface of the petri dish are peeled off using a cell scraper, and an immature dendritic cell suspension is recovered in a centrifuge tube. After centrifugation at 4°C and 500 g for 5 minutes, the supernatant is removed and an AIM medium is added. The cells in the plurality of petri dishes are put together in about 40 ml in one centrifuge tube. The number of cells in the immature dendritic cell suspension is measured.

The immature dendritic cell suspension is centrifuged, the supernatant is discarded, and a maturation medium (AIM medium with Picibanil at a final concentration of 10 µg/ml, PGE2 at a final concentration of 50 ng/ml, and neoantigen peptide at a final concentration of 20 µg/ml) is added to attain a final cell concentration of 1 to 2 × 10⁷ cells/6 ml. Each 6 to 12 ml of the cell suspension is placed in a petri dish and cultured in the incubator for 16 to 48 hours for induction of the cells into mature dendritic cells.

### (4) Recovery and cryopreservation of mature dendritic cell

The culture surface of the petri dish of the mature dendritic cells is pipetted using the culture supernatant. Thereafter, the culture solution containing the cells is recovered in the centrifuge tube. 10 ml of AIM medium is added to the petri dish, and the culture surface is pipetted to recover the AIM medium in a centrifuge tube. This procedure is repeated twice, and then a suspension of the mature dendritic cells is centrifuged at 4°C and 500 G for 5 minutes to remove the supernatant. 20 ml of AIM medium is added and recovered in a centrifuge tube set with a cell strainer. After measurement of the number of cells, the centrifuge tube is centrifuged at 4°C and 500 G for 5 minutes to remove the supernatant. The cells are suspended in a cell banker solution so that the final cell concentration is 1 × 10⁷ cells/ml, and dispensed into a cryotube. The cryotube is stored in the deep freezer at -80°C or a liquid nitrogen tank until the patient is inoculated with the mature dendritic cells.

### (Example 3: Isolation of CTC)

A cryopreserved monocyte-free PBMC (PBMC Mono-) fraction is promptly transported to an analysis facility with a zipper containing dry ice. In the analysis facility, the PBMC Mono- fraction transported in a frozen state is stored in a deep freezer or a liquid nitrogen tank until the PBMC Mono- fraction is analyzed.

In the analysis, the frozen cells are first dissolved at 37°C, and aggregated cells are removed using a filter. The remaining isolated cells are floated in 3 ml of PBS buffer, and the number of cells is measured. One or more monoclonal antibodies suitable for carcinoma or sarcoma are selected as the monoclonal antibody used in CTC collection, including an anti-EpCAM antibody (epithelial cell marker), an anti-Vimentin antibody, an anti-N-Cadherin antibody (epithelial-mesenchymal transition (EMT) marker), and an anti-CD20 antibody (malignant lymphoma marker). The CD45 antibody is used for control lymphocytes. Each antibody is labeled with a fluorescent substance including FITC or PE. The antibody is allowed to act on the CTCs at a concentration of 5 to 10 µg/ml (0.1 to 100 µg). The CTCs and control cells (CD45-positive cells) are then sorted using a cell sorter (SONY SH800).

### Example of sorting

| | |
|---|---|
| Number of cells of sorting source: | 1 × 10⁷ cells |
| PBS Buffer (2 mM EDTA, 2% FBS) | 485 µl |
| CD45 PE (200 µg/ml) | 10 µl |
| EpCAM FITC (1 mg/ml) | 5 µl |
| | 500 µl |

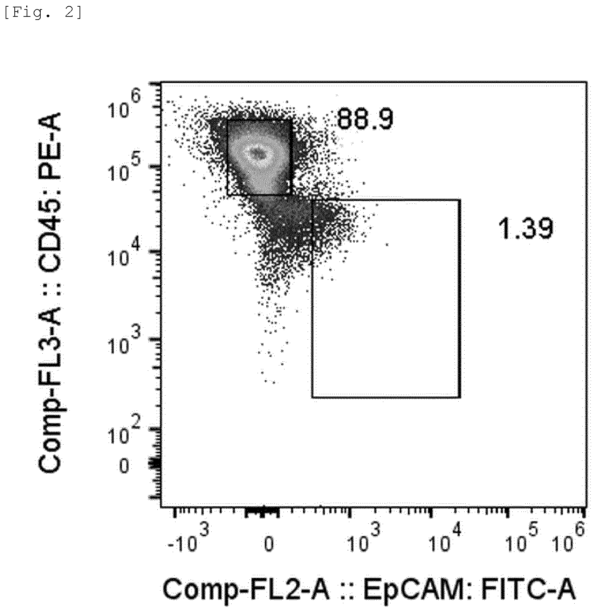

The collected cells are shown in Table 1 and FIG. 2 below.

**[Table 1]**

| Fraction | Number of cells collected |
|---|---|
| EpCAM | 29000 |
| CD45 | 1 × 10⁶ |

DNA extraction was performed as follows.

DNA extraction with QIamp (registered trademark) DNA Mini

Place 20 µl Proteinase K in a 1.5 ml microtube. Add the sample suspended in 200 µl PBS to this tube. Further add 200 µl Buffer AL and sufficiently mix by vortexing. Incubate at 56°C for 10 minutes, then spin down to recover the solution attached to the inside of the lid. Add 200 µl of 100% ethanol and sufficiently mix by vortexing. Add the sample to the QIamp Mini Spin Column, close the lid, and centrifuge at 6,000 g for 1 min. Discard the filtrate and the collection tube and attach a new collection tube. Add 500 µl Buffer AW1. Close the lid and centrifuge at 6,000 g for 1 min. Add 500 µl Buffer AW2. Close the lid and centrifuge at 12,000 g for 3 min. Discard the filtrate and centrifuge at 12,000 g for 1 min. Transfer QIamp Mini Spin Column to new 1.5 ml microtube. Add 100 µl Buffer AE and incubate at room temperature for 1 min. Centrifuge at 6,000 g for 1 min. Measure O.D and store the extracted DNA at -80°C.

| Sample ID | DNA concentration (ng/µl) | | volume (µl) | | Total amount of DNA (µg) | | OD260/80 | |
|---|---|---|---|---|---|---|---|---|
| 1-C-0062 | 6 | | 59 | | 0.354 | | 1.24 | |
| 2-C-0065 | 3 | | 60 | | 0.18 | | 1.57 | |
| 3-c-0052 | 1.3 | | 200 | | 0.26 | | 1.3 | |
| 4-c-0013 | 1.3 | | 200 | | 0.26 | | 1.04 | |

DNA can also be extracted with NucleoSpin (registered trademark) Blood L (TAKARA).

Each of the collected cell groups is centrifuged to remove the supernatant, and then suspended in 350 µl of an RLT buffer solution (1 ml of Buffer RLT Plus (QIAGEN) and 10 µl of β-ME (company name catalog number)).

### (Example 4: Extraction of RNA from CTC (RNeasy Mini Kit: QIAGEN))

An RLT buffer solution containing CTCs and CD 45+ cells is allowed to act on a gDNAEliminator spin column, and 350 µl of 70% ethanol is added to the solution collected by centrifugation. Thereafter, the mixture is caused to act on a Rneasy Min Elute spin column, and 700 µl of Buffer RW1 is added to the solution collected by centrifugation and centrifuged. Thereafter, the same treatment as with RW1 is performed with 500 µl of Buffer RPE, 500 µl of 80% ethanol, and 14 µl of Rnase-free water, and RNA is finally extracted. The amount of RNA is measured using NANO DROP ONE (Thermo SCIENTIFIC).

Examples of the extracted RNA are shown in Table 2 below.

**[Table 2]**

| sample name | Residual vol. (ul) | Bioanalyzer RNA Pico | | |
|---|---|---|---|---|
| sample ID | volume (ul) | RIN | cone (pg/ul) | amount (ng) |
| R1133-001_01 | 16 | 7.8 | 673 | 10.8 |
| R1133-002 | 15.5 | 8.1 | 6,489 | 100.6 |
| R1133-003 | 16.5 | 7.1 | 11,638 | 192 |
| R1133-001_02 | 13 | 6.9 | 5,803 | 75.4 |
| R1133-005 | 13 | 3.3 | 632 | 8.2 |
| R1133-006 | 14 | 8.8 | 34,671 | 485.4 |

The RNA extraction can be performed by using, in addition to the above method, kiazole or triazole. DNA can also be extracted from CTCs by the following method.

The whole exome analysis of DNA extracted from CTCs was performed as follows.

```
           Sample (total DNA)
           ↓
          Enrichment of exon region, and sequence library creation (SureSelect All
          Exon from Agilent technologies, exome kit from IDT xGen)
           ↓
           Sequencing (NovaSeq 6000 system, Illumina, Inc.)
           ↓
          Mapping (GEM, Subread, HISAT/HISAT2, KART)
           ↓
           Variant call (GATAC)
           ↓
          Result (creation of vcf file)
```

A library for whole exome sequence analysis is created from total DNA of CTCs and control cells using SureSelect All Exon kit from Agilent technologies. Quality inspection of the created sequence library is performed using TapeStation from Agilent. As a result of the quality inspection, it is confirmed that the sequence library has been created without any problem.

Sequencing is then performed by NovaSeq 6000 from Illumina, Inc.

Subsequently, mapping, expression difference analysis, and variant call are performed. Reads obtained by sequence are mapped using GEM (Marco-Sola, et al., 2012). Mapping can be performed by Subread (Liao, et al., 2013), HISAT/HISAT2 (Kim, et al., 2015), or KART (Lin and Hsu, 2017). Next, the number of reads mapped by HTSeq is counted, and gene expression analysis is performed. From map results, variant call is performed using The Genome Analysis Toolkit (GATK) "HaplotypeCaller" (Version: 4.0.10.1) to create a vcf file.

### (Example 5: Sequence of RNA extracted from CTC)

```
           • Flow of RNA sequence
           Sample (total RNA)
           ↓
           Sequence library creation (NEB rRNA depletion and TruSeq Stranded mRNA
           library) ↓
           Sequencing (HiSeq2500, 50-base paired-end)
           ↓
          Read filtering and trimming (moirai)
           ↓
          Mapping (STAR)
           ↓
          Mapping (HTSeq, egdeR)
           ↓
           Variant call (GATK HaplotypeCaller)
           ↓
          Result (creation of vcf file)
```

The ribosomal RNA is removed from the total RNA of the CTCs and the control cells using an rRNA depletion kit from NEB, and then an RNA-Seq library is created using TruSeq Stranded mRNA Sample Prep Kit from Illumina, Inc. Quality inspection of the created sequence library is performed using TapeStation from Agilent, followed by quantification by qPCR. As a result of the quality inspection, it is confirmed that the sequence library has been created without any problem, and a 5-plex RNA-Seq library is created.

Sequencing and base calling are then performed. A flow cell of HiSeq2500 Rapid Run Mode is used to sort reads for each index. It is confirmed that there is no problem in both Total yield and Q30.

Subsequently, mapping, expression difference analysis, and variant call are performed. The reads obtained in the sequence are mapped to the human standard sequence hg38 by a moirai pipeline with STAR (Hasegawa A et al, 2014). Next, the number of reads mapped by HTSeq is counted, and gene expression analysis is performed by edgeR. From map results, variant call is performed using The Genome Analysis Toolkit (GATK) "HaplotypeCaller" (Version: 4.0.10.1) to create a vcf file.

### (Example 6: Identification of neoantigen in silico)

Neoantigens are identified in silico by vcf files of the RNA sequences of CTCs and control cells, from which one is selected that is predicted to have a T cell response. Software including NetMHC, MHCflurry, OpenVax, and Neoantimon is used for analysis. The binding peptide structures of the selected neoantigens to HLA class I and class II are determined and their affinity for each HLA is predicted. Peptides with high affinity for HLA molecules are created (requested to peptide synthesis companies such as Cosmo Bio Co., Ltd. and Takara Bio Inc.) and administered to dendritic cells in II (3). The peptide to be administered may be any of HLA class I peptide, HLA class I peptide + class II peptide, and a long peptide including HLA class I and class II.

Neoantimon is an open program developed by The Institute of Medical Science, The University of Tokyo, which is software for identifying neoantigens specific for cancer cells in the environment (Mac/Linux). As an input file, a vcf file created by a mutation analysis program can be used, and it is possible to exhaustively calculate affinity between a mutated peptide fragment that can be generated from single base mutation (SNV), insertion/deletion (Indel), structural polymorphism (SV), and the like and HLA and to extract a peptide (neoantigen) having high antigen presentation ability (https://github.com/hase62/Neoantimon).

The identification results of neoantigens in colorectal cancer cases are shown in the following Tables 3 and 4.

### (1) Somatic mutation

**[Table 3]**

| Type | Number of mutations |
|---|---|
| Somatic SNVs | 1,501 |
| Nonsynonymous SNVs | 123 |
| Somatic INDELs | 91 |
| Exonic INDELs | 22 |

### (2) Identified neoantigens (SEQ ID NOs: 1 to 22, in order from the top, for the sequences set forth in the column "Neoantigen")

**[Table 4]**

| HLA | Chr | Position | Reference | Variant | Neoantigen | Presentation | Affinity | Gene | AA change | Type |
|---|---|---|---|---|---|---|---|---|---|---|
| HLA-C0303 | chr6 | 37012655 | T | C | MAAVGVSSV | 0.13948951 | 9.6 | COX6A1P2 | F7S | SNV |
| HLA-A2402 | chr12 | 10467915 | A | G | RYIYRVLL | 0.1215 | 9.66029177 | KLRD1 | K42R | SNV |
| HLA-A2402 | chr1 | 1.04E+08 | G | GA | PWADFSFKF | 0.837517659 | 11.2083545 | RNPC3 | NA | INDEL |
| HLA-A0201 | chr1 | 1.04E+08 | G | GA | FSFKFMPQV | 0.401501534 | 12.4832544 | RNPC3 | NA | INDEL |
| HLA-A2402 | chr1 | 1.04E+08 | G | GA | TWLFNSRKW | 0.504798904 | 12.7085048 | RNPC3 | NA | INDEL |
| HLA-A0201 | chr6 | 1.17E+08 | A | C | AQWGHVHSL | 0.583494153 | 13.2851856 | DSE | K81Q | SNV |
| HLA-A2402 | chr14 | 1.07E+08 | C | T | TFSNYGMHW | 0.306768375 | 17.3262681 | IGHV3-33 | S50N | SNV |
| HLA-C0303 | chr1 | 2.26E+08 | A | G | MAHCILDLL | 0.1003755 | 35.2054581 | SRP9 | 164M | SNV |
| HLA-A0201 | chr1 | 2.35E+08 | G | GA | TLKELFFRL | 0.704782469 | 37.0695827 | ARID4B | NA | INDEL |
| HLA-A0201 | chr1 | 1.04E+08 | G | GA | FMPQVYVPTT | 0.158649154 | 42.4514415 | RNPC3 | NA | INDEL |
| HLA-A0201 | chr1 | 1.5E+08 | G | GA | CLCPHPPAA | 0.122760697 | 47.5556603 | PLEKHO1 | NA | INDEL |
| HLA-C0303 | chr1 | 1.04E+08 | G | GA | WADFSFKFM | 0.712384294 | 51.3582243 | RNPC3 | NA | INDEL |
| HLA-C0303 | chr6 | 1.17E+08 | A | C | AQWGHVHSL | 0.236685111 | 52.629205 | DSE | K81Q | SNV |
| HLA-A0201 | chr1 | 1.04E+08 | G | GA | FMPQVYVPT | 0.180866579 | 56.34 | RNPC3 | NA | INDEL |
| HLA-A0201 | chr11 | 11373900 | C | T | FLGTEDLYDYI | 0.112389471 | 72.7 | CSNK2A3 | G256D | SNV |
| HLA-A0201 | chr20 | 36147533 | T | C | LLIPRPLNPA | 0.370699497 | 72.7599057 | BLCAP | K15R | SNV |
| HLA-A0201 | chr20 | 36147533 | T | C | LLIPRPLNPAL | o.759119592 | 75.319946 | BLCAP | K15R | SNV |
| HLA-A0201 | chr1 | 2.35E+08 | G | GA | KTLKELFFRL | 0.159787687 | 77.4668759 | ARID4B | NA | INDEL |
| HLA-A2402 | chr1 | 1.04E+08 | G | GA | FMPQVYVPTTF | 0.451739003 | 80.3138474 | RNPC3 | NA | INDEL |
| HLA-C0303 | chr14 | 1.07E+08 | C | T | FSNYGMHWV | 0.265815327 | 84.6737646 | IGHV3-33 | S50N | SNV |
| HLA-C0303 | chr1 | 1.04E+08 | G | GA | FSFKFMPQV | 0.339198911 | 96.8 | RNPC3 | NA | INDEL |
| HLA-A0201 | chr1 | 1.5E+08 | G | GA | HLHPPGTQEV | 0.573128904 | 97.3330371 | PLEKHO1 | NA | INDEL |

### (Example 7: Flow to production of pulsed cell)

A neoantigen-DC production step flow will be shown below.

### (A) In case of short peptide

### 5. Preparation of freezing medium and dispensing of reagents

### 5.1. Preparation of freezing medium

5.1.1. Based on the total number of cells in DCM, the required amount of a freezing medium and the amount of each of the a medium, DMSO, and albumin used are calculated.
5.1.2. Weigh a calculated amount of a medium in a centrifuge tube.
5.1.3. Add a calculated amount of DMSO to the weighed medium, and mix the mixture by inversion five or more times.
5.1.4. Add a calculated amount of albumin to the mixture in 5.1.3., and mix the mixture by inversion five or more times.
5.1.5. Weigh a medium in an equal amount to that of the prepared freezing medium into a centrifuge tube.
5.1.6. Store the freezing medium and the medium in a cold storage set at 4°C until just before use.

### 5.2. DMSO Dispensing

DMSO is dispensed only when an antigen that is dissolved in DMSO is used. However, this operation is unnecessary when an antigen already dissolved in DMSO (dispensed or the like) is used at the same time.
5.2.1. Use a syringe equipped with an injection needle to weight about 0.5 mL of DMSO into a sample tube.
5.2.2. Store it at room temperature until just before use.

### 5.3. Physiological saline and distilled water dispensing

Dispensing is performed only when an antigen that is dissolved in physiological saline and distilled water is used. However, this operation is unnecessary when a dispensed product is used.
5.3.1. Wipe lids of bottles of physiological saline and distilled water soluble with an alcohol swab and open them.
5.3.2. Weigh arbitrary amounts of physiological saline and distilled water into a sample tube.
5.3.2. Store them in a cold storage set at 4°C until just before use.

### 6. Procedure

### 6.1. Mature dendritic cell recovery

6.1.1. Confirm that the elapsed time from the end of DCM is 12 hours or more and 36 hours or less.
6.1.2. Take out petri dishes from an incubator and carry them into a work area. *One of the petri dishes or a petri dish for observation is inspected under a microscope. Discard the inspected petri dish for observation.
*In principle, five petri dishes are handled as one set, and the following operations in 6.1.3. to 6.1.8. are performed on all the petri dishes for each set.
6.1.3. Use a pipette to recover the cells in each of the petri dishes together with the culture supernatant in a 50 mL centrifuge tube.

Place cells pulsed with each antigen during DCM and cells not pulsed in separate centrifuge tubes.

(In the subsequent operations, different centrifuge tubes are used for the respective cells so as to prevent mixing.)
6.1.4. Use a pet to evenly wash the bottom surface of the petri dish with 5 to 10 mL of the culture medium, and recover the cells in a 50 mL centrifuge tube.
6.1.5. Use a pipette to dispense 1 to 2 mL of the medium into the petri dish.
6.1.6. Evenly apply a cell scraper to the bottom surface of the petri dish.
6.1.7. Use a pipette to recover the cell suspension from the petri dish in a 50 mL centrifuge tube.
6.1.8. Use a pipette to evenly wash the bottom surface of the petri dish with 5 to 10 mL of the culture medium, and recover the cells in a 50 mL centrifuge tube.
6.1.9. Perform the operation in 6.1.8. above a required number of times.
6.1.10. Centrifuge.
   Setting conditions: 500 G 5 min 4°C
6.1.11. If there is an antigen pulse, proceed to the procedure "6.2. Antigen pulse", and if there is no antigen pulse, proceed to the procedure "6.3. Cell freezing".
6.2. Antigen pulse
   Apply an antigen pulse only to cells that has not been pulsed during DCM.
6.2.1. After completion of the centrifugation, remove the supernatant.
6.2.2. Break pellets by tapping.
6.2.3. Use a pipette to gather the respective cells in the centrifuge tubes, by means of 5 to 20 mL of a medium, in one 50 mL centrifuge tube (hereinafter "centrifuge tube 1").
*If the centrifuge does not have a temperature regulating function, the use of a low-adhesion centrifuge tube is recommended as the centrifuge tube 1.
6.2.4. Gather the cells remaining in the centrifuge tubes after cell recovery in the centrifuge tube 1 while washing the centrifuge tubes with a medium, and adjust the volume to about 40 mL. (Cell suspension 1)
*The liquid volume is measured for calculating the number of cells.
6.2.5. After 5 or more times of mixing by inversion, collect 50 µL of the mixture in a microtube using a micropipette.
6.2.6. Dilute the suspension 2 to 20 times with a trypan blue stain, and count the number of living cells and the number of dead cells using a hemocytometer.
6.2.7. Calculate the total number of living cells from the count number.
6.2.8. Based on the calculated total number of cells, calculate the amount of each of the medium and the antigen used.
6.2.9. When pulsing the cells with the antigen, dissolve the antigen in a specified solvent so as to attain 2 mg/mL.
6.2.10. Weigh a calculated amount of a medium in a centrifuge tube. (The number of types of antigens to be used are prepared.)
6.2.11. Collect about 5 mL from each weighed medium, and dissolve a calculated amount of each antigen.
6.2.12. Pass the solution in 6.2.11 through a syringe filter and add it to the medium in 6.2.10 (hereinafter referred to as "peptide-added medium").
6.2.13. Dispense the cell suspension 1 into centrifuge tubes corresponding to the number of types of antigens used, followed by centrifugation.
   Setting conditions: 500 G 5 min 4°C
6.2.14. After completion of the centrifugation, recover the supernatant in a 50 mL test tube.
6.2.15. Collect 1 mL of the recovered supernatant in a sample tube and use it as a step reference product.
   (The supernatant may be collected from each centrifuge tube and collected into one tube.)
6.2.16. Break pellets by tapping, add each peptide-added medium thereto, and suspend the pellet therein.
6.2.17. Place the suspension in an incubator and allow it to stand for 30 to 60 minutes.
   Setting conditions: 37°C
6.2.18. Centrifuge.
   Setting conditions: 500 G 5 min 4°C
6.3. Cell freezing
6.3.1. After completion of the centrifugation, remove the supernatant.
6.3.2. Remove pellets by tapping.
6.3.3. Use a pipette to gather the respective cells in a 50 mL centrifuge tube set with a cell strainer while washing them with a medium, and adjust the volume to about 40 mL (hereinafter, "cell suspension 2").
*The liquid volume is measured for calculating the number of cells.
6.3.4. After 5 or more times of mixing by inversion, collect 50 µL of the cell suspension 2 in a microtube using a micropipette.
6.3.5. Dilute the suspension 2 to 20 times with a trypan blue stain, and count the number of living cells and the number of dead cells using a hemocytometer.
6.3.6. Calculate the number of living cells from the count number.
6.3.7. Based on the calculated total number of cells, calculate the number of frozen tubes and the amounts of the medium and freezing medium used.
6.3.8. Prepare the calculated number of tubes for frozen storage.
6.3.9. Centrifuge the cell suspension 2.
   Setting conditions: 500 G 5 min 4°C
6.3.10. After completion of the centrifugation, recover the supernatant in a 50 mL centrifuge tube.
6.3.11. Dispense the recovered supernatant for safety testing. (The supernatant may be collected from each centrifuge tube and collected into one tube.)
6.3.12. Break pellets by tapping.
6.3.13. Suspend the cells in the medium and match the amount of the medium to the "amount of the medium used" calculated in 6.3.7.
6.3.14. Collect an analyte for flow cytometry analysis and an analyte for product reference from vaccines having the largest number of cells. Use a micropipette to collect 0.1 mL of the suspension in 6.3.13 in each of two tubes for frozen storage, and add 0.4 mL of the medium and 0.5 mL of the freezing medium to each tube for suspension.
6.3.15. Add the freezing medium in the "amount of the freezing medium used" calculated in 6.3.7 to the suspension in 6.3.13, followed by gent pipetting to mix. 6.3.16. Perform foreign object inspection.
6.3.17. Dispense the whole amount by 1 mL into the tubes for frozen storage.
6.3.18. Place each of the tubes for frozen storage in Bicell and allow it to stand in a freezer set at -80°C.
6.3.19. Allow the Bicell to stand in a freezer set at -80°C, and transfer then frozen cells to a freezer set at -135°C or lower or a liquid nitrogen storage container within 3 hours or more and 36 hours or less.

### (B) In case of long peptide

### 5. Reagent dispensing

### 5.1. DMSO Dispensing

### Dispense DMSO as necessary.

5.1.1. Use a syringe equipped with an injection needle to weight an appropriate amount of DMSO into a sample tube.
5.1.2. Store it at room temperature until just before use.
5.2. Distilled water and physiological saline dispensing
   Dispense distilled water or physiological saline as necessary.
5.2.1. Weigh an appropriate amount of distilled water or physiological saline into a sample tube.
5.2.2. Store them in a cold storage set at 4°C until just before use.

### 6. Procedure

### 6.1. Immature dendritic cell recovery

6.1.1. Take out petri dishes from an incubator and carry them into a work area.
*One of the petri dishes or a petri dish for observation is inspected under a microscope. Discard the inspected petri dish for observation.
*In principle, five petri dishes are handled as one set, and the following operations in 6.1.2. to 6.1.8. are performed on all the petri dishes for each set.
6.1.2. Use a pipette to recover the cells in each of the petri dishes together with the culture supernatant in a 50 mL centrifuge tube.
6.1.3. Use a pipette to evenly wash the bottom surface of the petri dish with 5 to 10 mL of the culture medium, and recover the cells in a 50 mL centrifuge tube.
6.1.4. Use a pipette to dispense 1 to 2 mL of the medium into the petri dish.
6.1.5. Evenly apply a cell scraper to the bottom surface of the petri dish.
6.1.6. Use a pipette to recover the cell suspension from the petri dish in a 50 mL centrifuge tube.
6.1.7. Use a pipette to evenly wash the bottom surface of the petri dish with 5 to 10 mL of the culture medium, and recover the cells in a 50 mL centrifuge tube.
6.1.8. Perform the operation in 6.1.7. above a required number of times.
6.1.9. Centrifuge.
   Setting conditions: 500 G 5 min 4°C
6.1.10. After completion of centrifugation, remove the supernatant, and break pellets by tapping.
6.1.11. Use a pipette to gather the cells in the respective centrifuge tubes, by means of 5 to 20 mL of a medium, in one 50 mL centrifuge tube (hereinafter "centrifuge tube 1").
6.1.12. Gather the cells remaining in the respective centrifuge tubes after cell recovery in the centrifuge tube 1 while washing the centrifuge tubes with a medium, and adjust the volume to about 40 mL (hereinafter "cell suspension 1").
   *The liquid volume is measured for calculating the number of cells.
6.2. Cell number measurement
6.2.1. After 5 or more times of mixing by inversion, collect 50 µL of the cell suspension 1 in a microtube using a micropipette.
6.2.2. Dilute the suspension 2 to 20 times with a trypan blue stain, and count the number of living cells and the number of dead cells using a hemocytometer.
6.2.3. Calculate the total number of living cells from the count number.
6.2.4. Prepare the medium by the operation in the procedure "6.3. Medium preparation (without filtration)" when the predetermined reagent is not used, and prepare the medium by the operation in the procedure "6.4. Medium preparation (with filtration)" when the predetermined reagent is used. Perform the operation in the procedure "6.5. Cell seeding". In the case of PGE₂, filtration is performed only when unsterilized PGE₂ was used.
6.3. Medium preparation (without filtration)
6.3.1. Base on the calculated total number of living cells, calculate the number of petri dishes and the amounts of the medium, GM-CSF, IL-4, Picibanil, and PGE₂.
6.3.2. Weigh a calculated amount of a medium in a centrifuge tube.
6.3.3. Use a micropipette to add calculated amounts of GM-CSF, IL-4, Picibanil, and PGE₂ (hereinafter, "preparation medium").
6.4. Medium preparation (with filtration)
6.4.1. Base on the calculated total number of living cells, calculate the number of petri dishes and the amounts of the medium, GM-CSF, IL-4, Picibanil, PGE₂, and antigen used.
6.4.2. When antigen A is pulsed, dissolve antigen A in DMSO so as to attain 2 mg/mL.
6.4.3. When antigen B is pulsed, dissolve antigen B in distilled water or physiological saline so as to attain 2 mg/ml.
6.4.4. Weigh the medium in a centrifuge tube.
   *When two or more neoantigen peptides are used, the medium is appropriately weighed into separate centrifuge tubes under the instructions of a doctor, and the operations in
6.4.5. to 6.5.7. are performed for each centrifuge tube.
   In addition, the types of neoantigen peptide used in the present step and the next step are taken into consideration.
6.4.5. Collect about 5 mL from the weighed medium, and dissolve a calculated amount of each of antigen A and antigen B.
*When two or more antigens are used, each antigen is dispensed into a separate medium.
*When unsterilized PGE₂ is used, it is added in the present step.
6.4.6. Use a micropipette to add calculated amounts of GM-CSF, IL-4, Picibanil, and PGE₂ to the medium in 6.4.4.
6.4.7. Pass the solution in 6.4.5 through a syringe filter and add it to the medium in 6.4.6 (hereinafter referred to as "peptide-added medium").
6.5. Cell seeding
6.5.1. Prepare a calculated number of petri dishes.
6.5.2. Centrifuge a cell suspension.
   Setting conditions: 500 G 5 min 4°C
*The cell suspension is appropriately dispensed into an appropriate number of centrifuge tubes according to the type of neoantigen under the instructions of a doctor.
6.5.3. After completion of the centrifugation, recover the supernatant in a centrifuge tube.
6.5.4. Collect 1 mL of the recovered supernatant in a sample tube and use it as a step reference product.
6.5.5. Break pellets by tapping, add the preparation medium thereto, and suspend the pellet therein.
6.5.6. Use a pipette to dispense 6 mL of the medium into each of the petri dishes.
*In dispensing, pipetting is sufficiently performed.
*2 mL is dispensed into the petri dish for observation.
6.5.7. Place the petri dish in an incubator and allow it to stand overnight (12 hours or more and less than 36 hours).
   Setting conditions: 37°C, CO₂ concentration: 5.0%

As described above, neoantigen peptide pulsed dendritic cells are obtained.
• Final formulation step:
• Preparation of cell density (2 × 10⁷ cells/mL)
Suspend cells in a medium.
• Mixing with cell cryopreservation solution (1 × 10⁷ cells/mL)

Mix the cell suspension (medium) and a cell cryopreservation solution at 1:1.

The prepared concentration is high (2 times) so that the mixed solution will be diluted 2 times at the time of use.

It is also possible to cryopreserve 0.1 to 1 × 10⁷ cells/mL cells at 0.5 to 1 mL/tube.
• Filling into cryotube or the like (1 × 10⁷ cells/1 mL/tube)
Dispense the cell suspension into a cryotube.
• Labeling to cryotube (cryopreserved cell) and ampoule of attached thawing agent (physiological saline)
• Storage of formulation: cryopreserved cells (≤ -80°C)
Physiological saline (room temperature)
A final formulation (cryopreserved cells + attached thawing agent) is obtained.

In a method of pelleting the cells and adding a cell freezing agent as it is, the following operations are performed.
A: Suspend the cells in a cell cryopreservation solution (1 × 10⁷ cells/mL)
B: Dispense 1 mL of a cell suspension into a cryotube (1 × 10⁷ cells/1 mL/tube)

### (Example 8: Dendritic cell therapy)

Dendritic cell therapy is performed using the pulsed dendritic cells produced in Example 7.
1) Make a solution (thawing solution) by adding 8 mL of physiological saline to 1 mL of dextran.
2) Thaw the DCs to about 70% using a heat block obtained by heating a cryovial containing DCs (1 × 10⁷ cells/1 mL) frozen under liquid nitrogen to 37°C.
3) Dispense a portion of the thawing solution into a centrifuge tube, and transfer the DCs to a centrifuge tube (total amount: 10 ml) while rinsing the cryovial containing the DCs with the remaining thawing solution.
4) After mixing by inversion, collect 50 µL of the solution as a sample for counting and dilute it 2 times with a trypan blue stain. Count the number of living cells and the number of dead cells using a hemocytometer, and calculate the total number of cells and the living cell rate.
5) Centrifuge (500 g, 5 min, 4°C, with accelerator/brake). After completion of centrifugation, remove the supernatant, and break pellets by tapping. Add physiological saline to the cell suspension to attain about 13 mL. Repeat this operation twice.
6) Resuspend a frozen DC vaccine in about 0.6 to 0.8 mL of physiological saline and transfer it to a syringe (for insulin self-injection, 30 G × 10 mm, 0.5 mL).
7) Administer the vaccine subcutaneously to intradermally near axillary lymph nodes, near inguinal lymph nodes, or, when the lesion is close to the epidermis, in several places near the lesion.

### (Example 9: Proteome analysis)

In this example, after the CTCs are collected, the neoantigen protein expressed in the CTCs is identified by proteome analysis of the CTCs. The proteome analysis technique is as follows.
(1) A suspension containing about 1000 to tens of thousands of CTCs is centrifuged at 1000 to 2000 cpm for 10 to 15 minutes to make cell pellets. The cell pellets are stored in a low temperature tank at -80°C or a liquid nitrogen container.
(2) Proteins are extracted from the pellets of the CTCs by any method of surfactant, freezing-thawing, and osmotic shock.
(3) The extracted proteins are analyzed by an apparatus (liquid chromatograph mass spectrometer "Liquid Chromatograph-Mass Spectrometry: LC-MS/MS") in which a high performance liquid chromatograph (HPLC) and a triple quadrupole mass spectrometer (MS/MS) are combined. Thus, the proteins expressed in the CTCs are identified.

### (Example 10: Isolation of CTCs from various cancer patients)

CTCs were isolated using peripheral blood mononuclear cells from various cancer patients. PBMCs derived from patients with lung cancer (stage 4), colorectal cancer (stage 4), ovarian cancer (stage 4), breast cancer, pancreatic cancer (stage 4), duodenal papilla cancer (stage 4), colorectal cancer (stage 4), liver cancer, multiple liver cancer, prostate cancer (stage 4), and esophageal cancer as cancer patients were used. The techniques of collection of PBMCs, isolation of monocytes from PBMCs, and isolation of CTCs were performed in the same manners as in Examples 1 to 3.

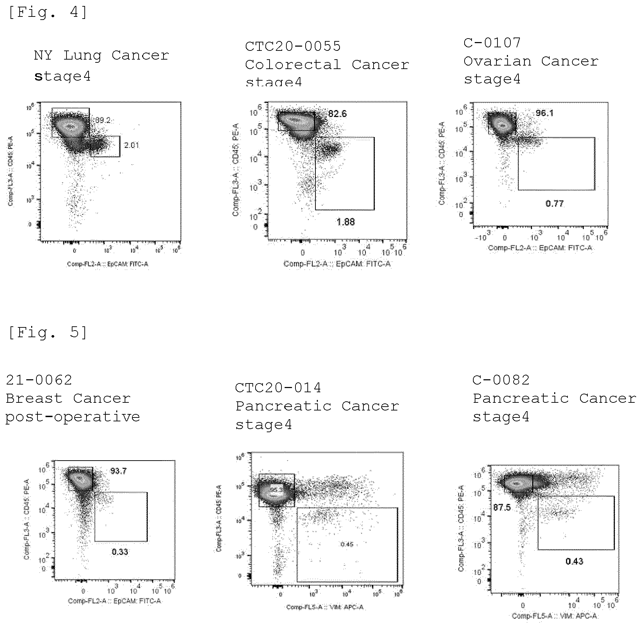

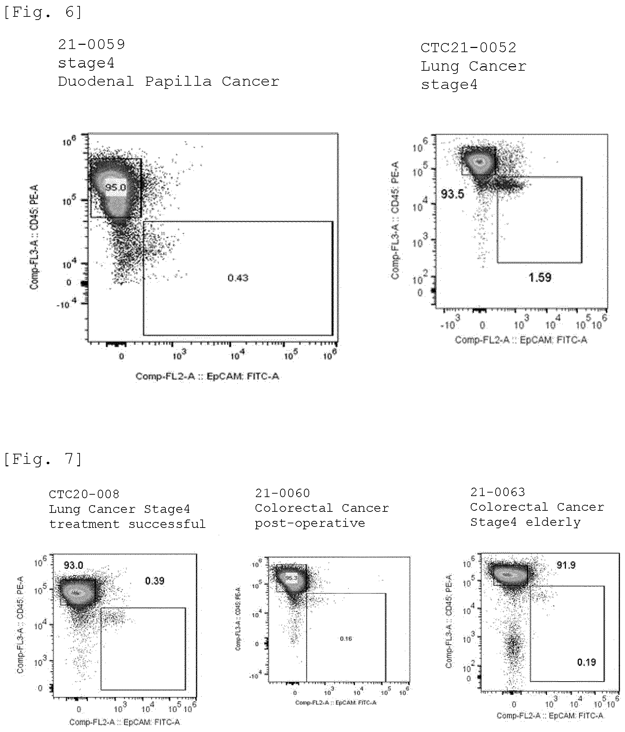

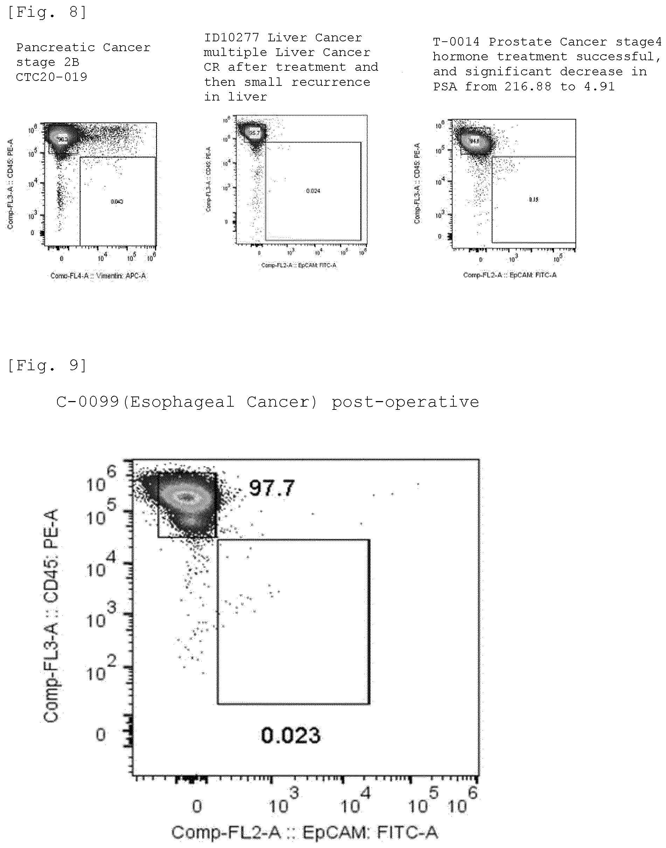

The results are shown in FIGS. 4 to 9. The analysis results of the flow cytometry shown in FIGS. 4 to 6 are for cases in which many CTCs are obtained, and the analysis results of the flow cytometry shown in FIGS. 7 to 9 are for cases in which the number of CTCs is small. In the case where the number of CTCs is small, the treatment is successful, or the cancer is completely excised by a surgical operation.

### (Example 11: Identification of neoantigen by RNA sequences or exome and proteome analyses)

The CTCs isolated from various cancer patients were used to identify neoantigens by RNA sequence or exome analysis. The results are shown in Tables 5 to 13 below. The RNA sequence was performed in the same manner as in Example 5. Tables 5 to 8 show the results of identification of neoantigens by exome analysis. By adding proteome analysis, it is possible to narrow down and identify only the neoantigens expressed as proteins. Narrow identification of neoantigens is also possible by combining RNA sequences with exome analysis, instead of proteome analysis. Tables 9 to 12 show the results of identification of neoantigens by RNA sequence.

**[Table 5]**

| Duodenal papilla cancer (DNA WES) WES : whole exome sequence | | | | | | | | | | | |
|---|---|---|---|---|---|---|---|---|---|---|---|
| **Chr** | **Position** | **Ref** | **Variant** | **HLA_Class1** | **Neoantigen** | **Presentation** | **Affinity** | **Gene** | | **AA_change** | **Type** |
| chr18 | 658064 | G | C | HLA-A31:01 | RTMRPLPGR | 0.716577906 | 5.4 | C18orf56 | | R62G | SNV |
| chr18 | 658064 | G | C | HLA-B07:02 | RPLPGRIEV | 0.7555046 | 8.32 | C18orf56 | | R62G | SNV |
| chr19 | 4847713 | A | G | HLA-B15:01 | LQLSQALSLM | 0.240915583 | 12.83 | PLIN3 | | V275A | SNV |
| chr11 | 68840160 | A | G | HLA-B15:01 | RQAMMEKVRSY | 0.582085256 | 31.2 | TPCN2 | | K376R | SNV |
| chr11 | 68840160 | A | G | HLA-A31:01 | KVQLDSSHR | 0.237151276 | 55.2 | TPCN2 | | K376R | SNV |
| chr18 | 658064 | G | C | HLA-B07:02 | RPLPGRIEVR | 0.122670572 | 77.72 | C18orf56 | | R62G | SNV |
| chr18 | 658064 | G | C | HLA-A31:01 | RTMRPLPGRI | 0.123166945 | 148.3 | C18orf56 | | R62G | SNV |
| chr19 | 4847713 | A | G | HLA-B15:01 | ALLQLSQAL | 0.338271042 | 152.02 | PLIN3 | | V263A | SNV |

| **Chr** | **Position** | **Ref** | **Variant** | **HLA_Class2** | **Neoantigen** | | **Affin (nM)** | **%Rank _BA** | **Gene** | **AA_change** | **Type** |
|---|---|---|---|---|---|---|---|---|---|---|---|
| chr11 | 68840160 | A | G | DRB1_1501 | RQAMMEKVRSYGSVLLSAEE | | 8.93 | 0.01 | TPCN2 | K376R | SNV |
| chr11 | 68840160 | A | G | DRB1_1501 | HRQAMMEKVRSYGSVLLSAE | | 9.16 | 0.01 | TPCN2 | K376R | SNV |
| chr11 | 68840160 | A | G | DRB1_1501 | SHRQAMMEKVRSYGSVLLSA | | 9.48 | 0.01 | TPCN2 | K376R | SNV |
| chr11 | 68840160 | A | G | DRB1_1501 | HRQAMMEKVRSYGSVLLSAEEFQKL | | 11.67 | 0.04 | TPCN2 | K376R | SNV |
| chr11 | 68840160 | A | G | DRB1_1501 | SHRQAMMEKVRSYGSVLLSAEEFQK | | 11.71 | 0.04 | TPCN2 | K376R | SNV |
| chr11 | 68840160 | A | G | DRB1_1501 | RQAMMEKVRSYGSVLLSAEEFQKL | | 11.75 | 0.04 | TPCN2 | K376R | SNV |
| chr11 | 68840160 | A | G | DRB1_1501 | HRQAMMEKVRSYGSVLLSAEEFQK | | 11.78 | 0.04 | TPCN2 | K376R | SNV |
| chr11 | 68840160 | A | G | DRB1_1501 | SSHRQAMMEKVRSYGSVLLSAEEFQ | | 11.8 | 0.04 | TPCN2 | K376R | SNV |

| | | | | | | | | | | | |
|---|---|---|---|---|---|---|---|---|---|---|---|
| Identified neoantigens (SEQ ID NOs: 23 to 38, in order from the top, for the sequences set forth in the column "Neoantigen") | | | | | | | | | | | |

**[Table 6]**

| Lung cancer 1 (DNA WES) | | | | | | | | | | |
|---|---|---|---|---|---|---|---|---|---|---|
| **Chr** | **Position** | **Ref** | **Variant** | **HLA_Class1** | **Neoantigen** | **Presentation** | **Affinity** | **Gene** | **AA_change** | **Type** |
| chr17 | 38520007 | C | A | HLA-C03:03 | AAQERRLQL | 0.55068671 | 23.64 | CTD | H273Q | SNV |
| chr11 | 1017294 | A | T | HLA-B15:01 | TTHPTHVPSF | 0.11455672 | 32.49 | MUC6 | L1836H | SNV |
| chr17 | 38520007 | C | A | HLA-A31:01 | QLQSPLLGR | 0.666748396 | 117.50 | GJD3 | V21L | SNV |
| chrl9 | 34712618 | G | A | HLA-A31:01 | GQEFADFEYR | 0.620137081 | 119.11 | LSM14A | R448Q | SNV |
| chr17 | 38520007 | C | A | HLA-A31:01 | VQLQSPLLGR | 0.261792234 | 292.60 | GJD3 | V21L | SNV |

| **Chr** | **Position** | **Ref** | **Variant** | **HLA_Class2** | **Neoantigen** | **Affin (nM)** | **%Rank_ BA** | **Gene** | **AA_change** | **Type** |
|---|---|---|---|---|---|---|---|---|---|---|
| chr17 | 38520007 | C | A | DRB4_0101 | LDAVQLQSPLLGRLW | 23.62 | 0.01 | CTD-2267D19.3 | V21L | SNV |
| chr17 | 38520007 | C | A | DRB4_0101 | LLDAVQLQSPLLGRL | 24.8 | 0.02 | CTD-2267019.3 | V21L | SNV |
| chr17 | 38520007 | C | A | DRB4_0101 | SLLDAVQLQSPLLGRLW | 31.4 | 0.03 | CTD-2267D19.3 | V21L | SNV |
| chr17 | 38520007 | C | A | DRB4_0101 | LLDAVQLQSPLLGRLWL | 31.78 | 0.04 | CTD-2267D19.3 | V21L | SNV |
| chr17 | 38520007 | C | A | DRB4_0101 | DAVQLQSPLLGRLWL | 32.75 | 0.05 | CTD-2267D19.3 | V21L | SNV |

| | | | | | | | | | | |
|---|---|---|---|---|---|---|---|---|---|---|
| Identified neoantigens (SEQ ID NOs: 39 to 48, in order from the top, for the sequences set forth in the column "Neoantigen") | | | | | | | | | | |

**[Table 7]**

| Breast cancer (DNA WES) | | | | | | | | | | | |
|---|---|---|---|---|---|---|---|---|---|---|---|
| **Chr** | **Position** | **Ref** | **Variant** | **HLA**_**Class1** | **Neoantigen** | **Presentation** | **Affinity** | | **Gene** | **AA_change** | **Type** |
| chrl8 | 60382986 | CCGG... | C | HLA-B40:02 | REDRASAPAAL | 0.455302522 | 9.6963 | | PHLPP1 | NA | INDEL |
| chr16 | 778927 | TG | T | HLA-B40:02 | REQASGWGGL | 0.162129803 | 19.583 | | HAGHL | NA | INDEL |
| chr2 | 241696821 | C | G | HLA-A02:06 | LQDDVFPEH | 0.107939547 | 35.201 | | KIF1A | E925Q | SNV |
| chr19 | 1753737 | G | A | HLA-B40:02 | GELLSPSHA | 0.121810811 | 58.421 | | ONECUT3 | G26S | SNV |
| chr2 | 241696821 | C | G | HLA-A02:06 | LQDDVFPEHAL | 0.220825078 | 70.258 | | KIF1A | E925Q | SNV |
| chr11 | 61313642 | G | C | HLA-A02:01 | RLGEKPAAA | 0.349188634 | 86.048 | | SYT7 | V102A | SNV |
| chr11 | 61313642 | G | C | HLA-A02:01 | RLGEKPAAA | 0.349188634 | 86.048 | | SYT7 | V102A | SNV |
| chr11 | 1017363 | C | G | HLA-B15:02 | STAPVTATSF | 0.504452201 | 103.49 | | MUC6 | G1813A | SNV |

| **Chr** | **Position** | **Ref** | **Variant** | **HLA_Class2** | **Neoantigen** | | **Affin (nM)** | **%Rank _BA** | **Gene** | **AA_change** | **Type** |
|---|---|---|---|---|---|---|---|---|---|---|---|
| chr20 | 44258532 | A | T | DRB4_0101 | GGYRDKMRMQKTQLSPEIKV | | 12.31 | 0 | WFDC10A | K27M | SNV |
| chr20 | 44258532 | A | T | DRB4_0101 | QGGYRDKMRMQKTQLSPEIK | | 12.33 | 0 | WFDC10A | K27M | SNV |
| chr20 | 44258532 | A | T | DRB4_0101 | GYRDKMRMQKTQLSPEIKVC | | 12.45 | 0 | WFDC10A | K27M | SNV |
| chr20 | 44258532 | A | T | DRB4_0101 | AQGGYRDKMRMQKTQLSPEI | | 12.58 | 0 | WFDC10A | K27M | SNV |
| chr20 | 44258532 | A | T | DRB4_0101 | YRDKMRMQKTQLSPEIKVCQ | | 12.77 | 0 | WFDC10A | K27M | SNV |
| chr20 | 44258532 | A | T | DRB4_0101 | RDKMRMQKTQLSPEI | | 12.82 | 0 | WFDC10A | K27M | SNV |
| chr20 | 44258532 | A | T | DRB4_0101 | QAQGGYRDKMRMQKTQLSPEIKVCQ | | 12.96 | 0 | WFDC10A | K27M | SNV |
| chr20 | 44258532 | A | T | DRB4_0101 | LQAQGGYRDKMRMQKTQLSPEIKVC | | 12.97 | 0 | WFDC10A | K27M | SNV |

| | | | | | | | | | | | |
|---|---|---|---|---|---|---|---|---|---|---|---|
| Identified neoantigens (SEQ ID NOs: 49 to 64, in order from the top, for the sequences set forth in the column "Neoantigen") | | | | | | | | | | | |

**[Table 8]**

| Colorectal cancer (DNA WES) | | | | | | | | | | | |
|---|---|---|---|---|---|---|---|---|---|---|---|
| **Chr** | **Position** | **Ref** | **Variant** | **HLAClass I** | **Neoantigen** | **Presentation** | **Affinity** | **Gene** | | **AAchange** | **Type** |
| chr15 | 72978653 | T | C | **_** HLA-C01:02 | LCPMQRFL | 0.628674693 | 50000 | BBS4 | | **_** F29L | SNV |
| chr4 | 44682465 | T | C | HLA-A2402 | EFKEKPDMSRF | 0.240224685 | 50000 | GUF1 | | LSSP | SNV |
| chr15 | 72978653 | T | C | HLA-B46:01 | KLAGGLCPM | 0.189712755 | 50000 | BBS4 | | F29L | SNV |

| **Chr** | **Position** | **Ref** | **Variant** | **HLA_Class II** | **Neoantigen** | | **Affin (nM)** | **%Rank _BA** | **Gene** | **AA_change** | **Type** |
|---|---|---|---|---|---|---|---|---|---|---|---|
| chr10 | 129904745 | G | C | DRB1_1501 | KAAVGEEKDINTFLGTPVQKLDQPG | | 25.41 | 0.27 | MKI67 | P1427A | SNV |
| chr10 | 129904745 | G | C | DRB1_1501 | PKAAVGEEKDINTFLGTPVQKLDQP | | 25.56 | 0.28 | MKI67 | P1787A | SNV |
| chr10 | 129904745 | G | C | DRB1_1501 | TPKAAVGEEKDINTFLGTPVQKLDQ | | 26.01 | 0.29 | MKI67 | P1787A | SNV |
| chr10 | 129904745 | G | C | DRB1_1501 | AAVGEEKDINTFLGTPVQK | | 67.98 | 1.6 | MKI67 | P1787A | SNV |

| | | | | | | | | | | | |
|---|---|---|---|---|---|---|---|---|---|---|---|
| Identified neoantigens (SEQ ID NOs: 65 to 71, in order from the top, for the sequences set forth in the column "Neoantigen") | | | | | | | | | | | |

**[Table 9]**

| Pancreatic cancer 1 (RNA-seq) | | | | | | | | | | |
|---|---|---|---|---|---|---|---|---|---|---|
| **Chr** | **Position** | **Ref** | **Variant** | **HLA_Class1** | **Neoantigen** | **Presentation** | **Affinity** | **Gene** | **AA_change** | **Type** |
| chr14 | 106692088 | T | A | HLA-A02:06 | FLVAFLEGV | 0.69927478 | 1.805 | IGHV3 | I13F | SNV |
| chr2 | 89185388 | C | T | HLA-A02:06 | TQSSDSLAV | 0.156535483 | 20.43 | IGKV4 | P28S | SNV |
| chr14 | 106692088 | T | A | HLA-A02:06 | FLEGVQCEV | 0.251136338 | 21.34 | IGHV3 | I13F | SNV |
| chr12 | 56500857 | A | C | HLA-A02:06 | YILQEGDLV | 0.352475856 | 34.27 | PA2G4 | K90Q | SNV |
| chr14 | 106692088 | T | A | HLA-A24:02 | RWVFLVAFL | 0.180047357 | 41.56 | IGHV3 | I13F | SNV |
| chr12 | 56500857 | A | C | HLA-A02:06 | LQEGDLVKI | 0.530916473 | 52.97 | PA2G4 | K82Q | SNV |
| chr14 | 106725466 | C | T | HLA-A02:06 | QLLESGGDLV | 0.41728368 | 108.31 | IGHV3 | G29D | SNV |

| | | | | | | | | | | |
|---|---|---|---|---|---|---|---|---|---|---|
| Identified neoantigens (SEQ ID NOs: 72 to 78, in order from the top, for the sequences set forth in the column "Neoantigen") | | | | | | | | | | |

**[Table 10]**

| Pancreatic cancer 2 (RNA-seq) | | | | | | | | | | |
|---|---|---|---|---|---|---|---|---|---|---|
| **Chr** | **Position** | **Ref** | **Variant** | **HLA-Classl** | **Neoantigen** | **Presentation** | **Affinity** | **Gene** | **AA_change** | **Type** |
| chr2 | 89247012 | C | G | HLA-B58:01 | QSISTWLAW | 0.724901183 | 1.30 | IGKV1 | S53T | SNV |
| chr2 | 89247012 | C | G | HLA-B58:01 | RASQSISTW | 0.951657962 | 2.11 | IGKV1 | S53T | SNV |
| chr2 | 89247012 | C | G | HLA-B58:01 | ASQSISTWLAW | 0.554167579 | 16.55 | IGKV1 | S53T | SNV |
| chr7 | 150490376 | C | T | HLA-A02:06 | LTLTGFATA | 0.474076249 | 16.84 | TMEM176B | A134T | SNV |
| chr7 | 150490376 | C | T | HLA-B58:01 | ISSLLTLTGF | 0.250450077 | 17.96 | TMEM176B | A134T | SNV |
| chr7 | 150490376 | C | T | HLA-B58:01 | SSLLTLTGF | 0.487809122 | 79.34 | TMEM176B | A134T | SNV |
| chr7 | 150490376 | C | T | HLA-A02:06 | SLLTLTGFATA | 0.153920206 | 156.46 | TMEM176B | A134T | SNV |

| | | | | | | | | | | |
|---|---|---|---|---|---|---|---|---|---|---|
| Identified neoantigens (SEQ ID NOs: 79 to 85, in order from the top, for the sequences set forth in the column "Neoantigen") | | | | | | | | | | |

**[Table 11]**

| Pancreatic cancer 3 (RNA-seq) | | | | | | | | | | |
|---|---|---|---|---|---|---|---|---|---|---|
| **Chr** | **Position** | **Ref** | **Variant** | **HLA-Class1** | **Neoantigen** | **Presentation** | **Affinity** | **Gene** | **AA_change** | **Type** |
| chr1 | 40027426 | G | A | HLA-A11:01 | AVLQAHYAK | 0.879405078 | 15.21 | PABPC4 | H598Y | SNV |
| chr14 | 106641693 | T | C | HLA-A11:01 | SAYSGNTNY | 0.223560512 | 18.65 | IGHV1 | N74S | SNV |
| chr1 | 40027426 | G | A | HLA-A11:01 | AVAVLQAHYAK | 0.78962331 | 26.94 | PABPC4 | H643Y | SNV |
| chr1 | 40027426 | G | A | HLA-A11:01 | AVLQAHYAKK | 0.847122455 | 29.41 | PABPC4 | H643Y | SNV |
| chr1 | 40027426 | G | A | HLA-A31:01 | AVLQAHYAK | 0.140110099 | 42.44 | PABPC4 | H598Y | SNV |
| chr2 | 89185403 | G | A | HLA-A31:01 | SLAMSLGER | 0.318924839 | 71.54 | IGKV4 | V33M | SNV |
| chr1 | 40027426 | G | A | HLA-A11:01 | VLQAHYAKK | 0.453726321 | 75.71 | PABPC4 | H614Y | SNV |
| chr1 | 40027426 | G | A | HLA-A11:01 | VAVLQAHYAK | 0.223745531 | 82.71 | PABPC4 | H643Y | SNV |
| chr14 | 106610602 | A | T | HLA-A11:01 | QVESGGGLVK | 0.390891947 | 83.38 | IGHV3 | L23Q | SNV |
| chr14 | 106641693 | T | C | HLA-C14:02 | SAYSGNTNY | 0.115491904 | 89.25 | IGHV1 | N74S | SNV |
| chr14 | 106641693 | T | C | HLA-A11:01 | YSGNTNYAQK | 0.100446419 | 147.1 | IGHV1 | N74S | SNV |

| | | | | | | | | | | |
|---|---|---|---|---|---|---|---|---|---|---|
| Identified neoantigens (SEQ ID NOs: 86 to 96, in order from the top, for the sequences set forth in the column "Neoantigen") | | | | | | | | | | |

**[Table 12]**

| Lung cancer 2 (RNA-seq) | | | | | | | | | | |
|---|---|---|---|---|---|---|---|---|---|---|
| **Chr** | **Position** | **Ref** | **Variant** | **HLA-Class1** | **Neoantigen** | **Presentation** | **Affinity** | **Gene** | **AA_change** | **Type** |
| chr5 | 70858261 | G | A | HLA-A11:02 | RSNPFNESQK | 0.340464827 | 24.10 | BDP1 | E575K | SNV |
| chr5 | 70858261 | G | A | HLA-A11:02 | RSNPFNESQKK | 0.311006071 | 25.80 | BDP1 | E2553K | SNV |

| | | | | | | | | | | |
|---|---|---|---|---|---|---|---|---|---|---|
| Identified neoantigens (SEQ ID NOs: 97 to 98, in order from the top, for the sequences set forth in the column "Neoantigen") | | | | | | | | | | |

### (Example 12: Other analysis examples using CTC-diseases other than cancer)

Application examples to diseases other than cancer will be described.
(1) Gene analysis of CTCs has revealed various mutations of genes including driver genes, making it possible to select a molecular target drug.
(2) Also, it has become possible to detect high-frequency microsatellite instability (MSI-High) as in (1), and to identify whether it is an indication of an immune checkpoint inhibitor.
(3) By combining clinical data with transcriptome analysis or single-cell RNA-seq, it is possible to clarify the difference in CTCs between cases in which metastasis occurs (or is likely to occur) and cases in which metastasis does not occur (or is unlikely to occur). This leads to prediction of the prognosis of cases and elucidation of the mechanism by which metastasis occurs.
(4) By performing metabolome analysis (the metabolome analysis refers to comprehensively detecting all types and concentrations of metabolites present in a target sample and analyzing the results, and is also referred to as metabolomics), the metabolic characteristics of CTCs are clarified, and the reviews in (3) become possible more comprehensively.

### (Example 13: Other analysis examples using CTC - confirmation of health condition)

An application example in the case of confirming the health condition will be described.
(1) Exome analysis of CTCs can detect whether a malignancy is caused by a rare variant mutation. Furthermore, the presence of the same disease becomes clear by examining blood relatives in some cases, and early diagnosis and early treatment of the blood relatives become possible.
(2) When neoantigens are identified, DNA of not only CTCs but also normal cells can be extracted. The genome sequence of the normal germline can be determined by the whole genome analysis or the DNA chip analysis of the normal DNA. Based on this sequence, risk calculation of not only rare variant mutations related to diseases but also common variants becomes possible. It is also useful for preventive medicine for diseases other than malignancies.

### (Note)

Although the present disclosure has been illustrated using preferred embodiments of the present disclosure as above, it is understood that the scope of the present disclosure should be interpreted only by the claims. It is understood that the contents of patents, patent applications, and other documents cited herein are to be incorporated by reference herein in the same manner as if the contents thereof were specifically described herein. The present application claims priority to Japanese Patent Application No. 2021-108784 filed on June 30, 2021 to the Japanese Patent Office, the entire contents of which are incorporated herein by reference in the same manner as if the entire contents thereof constituted the contents of the present application.

### Industrial Applicability

The method of the present disclosure enables non-invasive identification of a neoantigen of a patient, and is thus useful for a trial of cancer immunotherapy using the neoantigen. In addition, the method can be expected to be developed into uses in regenerative medicine and uses to develop new medicine mainly focusing on cell therapy.

### Sequence Listing Free Text

SEQ ID NOs: 1 to 22: Neoantigens identified in Example 6
SEQ ID NOs: 23 to 98: Neoantigens identified in Example 11

## Claims

1. A method for preparing a sample containing circulating tumor cells derived from a subject for analysis without nucleic acid amplification and/or proliferation by culture, the method comprising the steps of:
isolating monocytes from peripheral blood mononuclear cells collected from the subject using apheresis, wherein an apheresis condition for performing the apheresis includes a circulating tumor cell enrichment condition;
isolating circulating tumor cells from the peripheral blood mononuclear cells from which the monocytes have been isolated;
optionally, confirming a purity of circulating tumor cells required for the analysis; and
optionally, performing pretreatment for the analysis on the circulating tumor cells.

2. The method according to claim 1, wherein the apheresis condition includes subjecting a spillover volume and/or a buffy coat volume to the circulating tumor cell enrichment condition.

3. The method according to claim 1 or 2, wherein the apheresis condition includes a spillover volume of about 9 to about 12 ml and/or a buffy coat volume of about 7 to about 10 ml.

4. The method according to any one of claims 1 to 3, wherein the step of isolating circulating tumor cells is carried out using a monoclonal antibody or by size fractionation.

5. The method according to any one of claims 1 to 4, wherein a number of the circulating tumor cells is about 1 × 10³ or more.

6. The method according to any one of claims 1 to 5, wherein a number of the peripheral blood mononuclear cells is about 1 × 10⁴ or more.

7. The method according to claim 4, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

8. The method according to any one of claims 1 to 7, further comprising a step of freezing the circulating tumor cells.

9. The method according to any one of claims 1 to 8, wherein the analysis includes whole exome analysis, whole genome analysis, RNA-Seq, single cell RNA-Seq, proteome analysis, and transcriptome analysis.

10. The method according to any one of claims 1 to 9, wherein the sample is for identifying a neoantigen possessed by the subject by performing the analysis.

11. The method according to any one of claims 1 to 10, further comprising a step of extracting DNA, RNA, or protein from the circulating tumor cells.

12. The method according to claim 11, wherein the DNA or RNA is extracted at least in an amount of about 100 pg.

13. A method for analyzing circulating tumor cells derived from a subject without nucleic acid amplification and/or proliferation by culture, the method comprising the steps of:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated; and
performing analysis using the circulating tumor cells.

14. The method according to claim 13, wherein the apheresis condition includes subjecting a spillover volume and/or a buffy coat volume to the circulating tumor cell enrichment condition.

15. The method according to claim 13 or 14, wherein the apheresis condition includes a spillover volume of about 9 to about 12 ml and/or a buffy coat volume of about 7 to about 10 ml.

16. The method according to any one of claims 13 to 15, wherein the step of isolating circulating tumor cells is carried out using a monoclonal antibody or by size fractionation.

17. The method according to any one of claims 13 to 16, wherein the number of the circulating tumor cells is about 1 × 10³ or more.

18. The method according to any one of claims 13 to 17, wherein the number of the peripheral blood mononuclear cells is about 1 × 10⁴ or more.

19. The method according to claim 16, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

20. The method according to any one of claims 13 to 19, further comprising a step of freezing the circulating tumor cells.

21. The method according to any one of claims 13 to 20, wherein the analysis includes whole exome analysis, whole genome analysis, RNA-Seq, single cell RNA-Seq, proteome analysis, and transcriptome analysis.

22. A method for identifying a neoantigen possessed by the subject by analyzing the circulating tumor cells derived from the subject by the method according to any one of claims 13 to 21.

23. The method according to claim 22, further comprising a step of extracting DNA, RNA, or protein from the circulating tumor cells.

24. The method according to claim 24, further comprising the steps of:
creating a DNA/RNA sequence library from the DNA or RNA; and
identifying a neoantigen based on mutation information in the sequence library,
wherein the circulating tumor cells have a purity of at least about 20%.

25. The method according to claim 24, wherein the analysis includes a whole exome analysis of the DNA.

26. The method according to claim 25, wherein the analysis further includes proteomic analysis of the circulating tumor cells.

27. The method according to any one of claims 23 to 26, wherein the DNA or RNA is extracted at least in an amount of about 100 pg.

28. A method for producing a cell for dendritic cell therapy using a neoantigen possessed by a subject, the method comprising:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
extracting DNA or RNA from the circulating tumor cell to create a DNA/RNA sequence library;
identifying a neoantigen based on mutation information of the sequence library; and
introducing the neoantigen into dendritic cells.

29. The method according to claim 28, wherein the apheresis condition includes subjecting a spillover volume and/or a buffy coat volume to the circulating tumor cell enrichment condition.

30. The method according to claim 28 or 29, wherein the apheresis condition includes a spillover volume of about 9 to about 12 ml and/or a buffy coat volume of about 7 to about 10 ml.

31. The method according to any one of claims 28 to 30, wherein the step of isolating circulating tumor cells is carried out using a monoclonal antibody or by size fractionation.

32. The method according to any one of claims 28 to 31, wherein a number of the circulating tumor cells is about 1 × 10³ or more.

33. The method according to any one of claims 28 to 32, wherein a number of the peripheral blood mononuclear cells is about 1 × 10⁴ or more.

34. The method according to claim 31, wherein the monoclonal antibody includes an anti-EpCAM antibody, an anti-Vimentin antibody, an anti-N-Cadherin antibody, an anti-CD20 antibody, an anti-E-Cadherin antibody, an anti-Desmoglein-3 antibody, an anti-Syndecan-1 antibody, an anti-CD99 antibody, an anti-CD81 antibody, and a PAX3 antibody.

35. The method according to any one of claims 28 to 34, further comprising a step of freezing the circulating tumor cells.

36. The method according to any one of claims 28 to 35, wherein the DNA or RNA is extracted at least in an amount of about 100 pg.

37. The method according to any one of claims 28 to 36, wherein the dendritic cells are obtained from the subject by the apheresis or derived from the monocytes.

38. A method for treating or preventing a subject by analyzing circulating tumor cells without nucleic acid amplification and/or proliferation by culture, the method comprising the steps of:
isolating monocytes from peripheral blood mononuclear cells collected from the subject using apheresis, wherein apheresis condition for performing the apheresis includes circulating tumor cell enrichment condition;
isolating circulating tumor cells from the peripheral blood mononuclear cells from which the monocytes have been isolated;
performing analysis using the circulating tumor cells; and
treating or preventing the subject based on results of the analysis.

39. The method according to claim 38, wherein the analysis includes whole exome analysis, whole genome analysis, RNA-Seq, single cell RNA-Seq, proteome analysis, and transcriptome analysis.
